# EUROPEAN PATENT SPECIFICATION

(11) **EP 1 140 925 B1**
(45) Date of publication and mention of the grant of the patent: **23.04.2003**
(21) Application number: 00902331.8
(22) Date of filing: 06.01.2000
(51) Int. Cl.: C07D 413/12, A61K 31/538, A61P 25/24

(54) **(3,4 DIHYDRO-2H-BENZO[1,4] OXAZINYL-METHYL)- 3-(1H-INDOL-3- YL)-ALKYL -AMINES**
(3,4-DIHYDRO-2H-BENZO[1,4]OXAZINYL-METHYL)-3-(1H-INDOL-3-YL)-ALKYLAMINE
3,4-DIHYDRO-2H-BENZO 1,4]OXAZINYL-METHYL 3-(1H-INDOL-3-YL)-ALKYL]-AMINES

(30) Priority: 07.01.1999 US 226831
(43) Date of publication of application: 10.10.2001
(73) Proprietor: Wyeth, Madison, New Jersey 07940-0874 (US)
(72) Inventor: MEWSHAW, Richard, Eric, King of Prussia, PA 19406 (US); ZHOU, Dahui, East Brunswick, NJ 08816 (US); ZHOU, Ping, Plainsboro, NJ 08536 (US)
(74) Representative: Wileman, David Francis Dr.
(86) International application number: US0000230
(87) International publication number: WO00040580

(56) References cited:
- WO-A-89/07596
- WO-A-99/51592
- A.-S. BOURLOT ET AL.: "New substituted 1,4-benzoxazine derivatives with potential intracellular calcium activity" JOURNAL OF MEDICINAL CHEMISTRY., vol. 41, no. 17, 1998, pages 3142-3158, XP002136635 AMERICAN CHEMICAL SOCIETY. WASHINGTON., US ISSN: 0022-2623

## Description

This invention is directed to compounds useful in the treatment of neurological diseases caused by disorders of the serotonin-affected neurological systems. More specifically, the present invention is directed to 3,4-dihydro-2H-benzo[1,4]oxazinyl-methyl)-[3-(lH-indol-3-yl)-alkyl]amines useful as anxiolytic and/or antidepressant agents, processes for their preparation and pharmaceutical compositions containing them.

### BACKGROUND OF INVENTION

Pharmaceuticals which enhance the neurotransmission of serotonin (5-HT) are useful for the treatment of many psychiatric disorders, including depression and anxiety. The first generation of non-selective serotonin-affecting drugs operated through a variety of physiological means which caused them to possess numerous undesired side-effects. The more recently prescribed drugs, the selective serotonin reuptake inhibitors (SSRIs), act predominately by inhibiting 5-HT, which is released at the synapses, from being actively removed from the synaptic cleft via a presynaptic serotonin transport carrier. Since SSRIs require several weeks before they exert their full therapeutic effect, this 5-HT blockade mechanism cannot fully account for their therapeutic activity. It is speculated that this two week induction which occurs before a full antidepressant effect is observed, is due to the involvement of the 5-HT1A autoreceptors which suppress the firing activity of 5-HT neurons, causing a dampening of the therapeutic effect. Studies suggest that after several weeks of SSRI administration, a desensitization of the 5-HT. autoreceptors occurs allowing a full antidepressant effect in most patients. (See, e.g., Le Poul et al., Arch. Pharmacol., 352:141 (1995)). Hence, it is believed that overriding this negative feedback by using 5HT1A antagonists would potentially increase and accelerate the clinical antidepressant response. Recent studies by Artigas et al., Trends Neurosci., 19:378-383 (1996), suggest a combination of 5-HT1A activity and inhibition of 5-HT uptake within a single molecular entity can achieve a more robust and fast-acting antidepressant effect.

The present invention relates to a new class of molecules which have the ability to act at the 5-HT1A autoreceptors and concommitantly with the 5-HT transporter. Such compounds are therefore potentially useful for the treatment of depression as well as other serotonin disorders.

Compounds having antidepressant and anxiolytic activity due to their affinity for the 5HT1A autoreceptors are disclosed in, e.g., U.S. Patent No. 3,717,634 which claims N-(heteroarcyclic)-piperazinylalkyl derivatives of azaspiroalkanediones. Aryl and heteroaryl piperazinylalkyl carboxamides having 5HT1A activity are disclosed, e.g., in U.S. Patent Nos. 5,106,849; 5,278,160; and 5,482,940.

Glutarimide derivatives having anxiolytic and antihypertensive activity are disclosed in U.S. Patent No. 4,612,312.

Benzoxazine and thiazine derivatives useful for treating psychotic disorders and schizophrenia are disclosed in PCT Patent Application Publication No. WO 8907596-A. However, none of these patents disclose compounds which act at both the 5HT1A autoreceptors and the 5-HT-transporter.

### SUMMARY OF INVENTION

The present invention is directed to compounds which are both active towards 5HT_{1A} receptors and inhibit 5HT transport. Such compounds are useful in treating disorders relating to serotinin concentrations, such as anxiety and depression.
The present invention is directed to compounds of the formula: 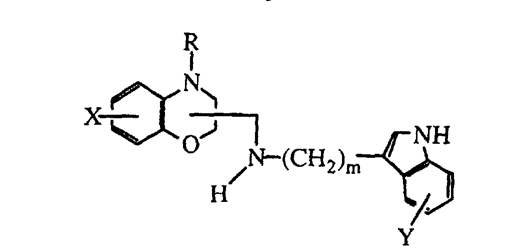 wherein:
R is hydrogen, alkyl of 1 to 6 carbon atoms, phenyl, benzyl or substituted phenyl;
X and Y are each, independently, hydrogen, halogen, cyano, or alkoxy of 1 to 6 carbon atoms; and
m is 1 to 5;or
a pharmaceutically acceptable salt thereof.

The present invention is further directed to methods for allevating the symptoms of depression comprising administering to a patient in need thereof an antidepressant effective amount of a compound represented by the following formula: 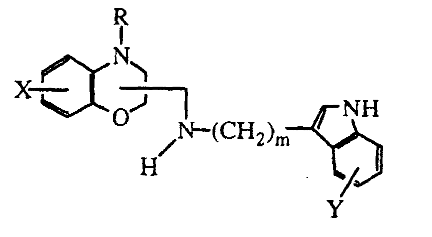 wherein:
R is hydrogen, alkyl of 1 to 6 carbon atoms, phenyl, benzyl, or substituted phenyl;
X and Y are each, independently, hydrogen, halogen, cyano, or alkoxy of I to 6 carbon atoms; and
m is 1 to 5; or
a pharmaceutically acceptable salt thereof.

### DETAILED DESCRIPTION OF THE INVENTION

Preferably, the present compounds are those represented by the formula set forth above wherein:
R is hydrogen or alkyl of 1 to 4 carbon atoms; and/or
X and Y are each, independently, hydrogen, halogen or cyano; and/or
m is 2 to 4; or
a pharmaceutically acceptable salt thereof.

Most preferably, the compounds of the present invention include:
(3,4)-Dihydro-2H-benzo[ 1,4]oxazine-2-yl-methyl)-[3-( 1 H-indol-3-yl)-propyl]-amine;
[3-(1H-Indol-3-yl)-propyl]-(4-methyl-3,4-dihydro-2H-benzo[1,4]oxazin-2-yl-methyl)-amine;
(4-Benzyl-3,4-dihydro-2H-benzo[1,4]oxazin-2-yl-methyl)-[3-(1H-indol-3-yl)-propyl]-amine;
(4-Benzyl-3,4-dihydro-2H-benzo[1,4]oxazin-2-yl-methyl)-[3-(1H-indol-3-yl)-butyl]-amine;
[3-(5-Fluoro-1H-indol-3-yl)-propyl]-(4-methyl-3,4-dihydro-2H-benzo[1,4]oxazin-2yl-methyl)-amine;
[3-(1H-Indol-3-yl)-propyl]-(4-benzyl-3,4-dihydro-2H-benzo[ 1,4]oxazin-2-yl-methyl)-amine;
[3-(1H-Indol-3-yl)-propyl]-(4-phenyl-3,4-dihydro-2H-benzo[1,4]oxazin-2-yl-methyl)- amine;
[3-(5-Fluoro-1H-indol-3-yl)-propyl]-(4-isopropyl-3,4-dihydro-2H-benzo[1,4]oxazin-2-yl-methyl)-amine;
[3-(1H-Indol-3-yl)-butyl]-(4-isopropyl-3,4-dihydro-2H-benzo[1,4]oxazin-2-yl-methyl)-amine;
[3-(5-Fluoro-1H-indol-3-yl)-propyl]-(4-isobutyl-3,4-dihydro-2H-benzo[1,4]oxazin-2-yl-methyl)-amine;
[3-(5-Fluoro-1H-indol-3-yl)-propyl]-(4-phenyl-3,4-dihydro-2H-benzo[1,4]oxazin-2-yl-methyl)-amine;
[3-(5-Fluoro-1H-indol-3-yl)-butyl]-(4-phenyl-3,4-dihydro-2H-benzo[1,4]oxazine-2-yl-methyl)-amine;
[3-(5-Fluoro-1H-indol-3-yl)-propyl]-(4-ethyl-3,4-dihydro-2H-benzo[1,4]oxazin-2-yl-methyl)-amine;
[3-(5-Fluoro-1H-indol-3-yl)-propyl]-(4-propyl-3,4-dihydro-2H-benzo[1,4]oxazine-2-yl-methyl)-amine;
(7-Chloro-4-methyl-3,4-dehydro-2H-benzo[1,4]oxazin-2-yl-methyl)-[3-5-fluoro-1H-indol-3-yl)-propyl]-propyl-amine;
(3,4-Dihydro-2H-benzo[1,4]oxazin-2-yl-methyl)-[3-(5-fluoro-1H-indol-3-yl)-propyl-amine;
(7-Methoxy-3,4-dihydro-2H-benzo[1,4]oxazin-2-yl-methyl)-[3-(5-fluoro-1H-indol-3-yl)-propyl]-amine;
[3-(5-Fluoro-1H-indol-3-yl)-propyl]-(4-phenyl-3,4-dihydro-2H-benzo[1,4]oxazin-2-yl-methyl)-amine; and
(3,4-dihydro-2H-benzo[1,4]oxazin-3-yl-methyl)-[3-(5-fluoro-1H-indol-3-yl)propyl]-amine.

As used herein, the terms "alkyl" and "alkoxy" are meant to include both straight and branched carbon chains of 1 to 6 carbon atoms, eg 1 to 4. The term "halogen" is meant to include fluorine, chlorine, bromine and iodine. The term "substituted phenyl" is meant to include a phenyl moiety substituted with an alkyl, a halogen, or alkoxy group.

The pharmaceutically acceptable salts of the present compounds are those derived from such inorganic cations such as sodium, potassium and the like; organic bases, such as mono-, di- and trialkyl amines of 1 to 6 carbon atoms, per alkyl group and mono-, di- and trihydroxyalkyl amines of 1-6 carbon atoms per alkyl group; and organic and inorganic acids, such as acetic, lactic, citric, tartaric, succinic, maleic, malonic, gluconic, hydrochloric, hydrobromic, phosphoric, nitric, sulfuric, methane sulfonic, and similarly known acceptable acids.

The compounds of the present invention may be prepared by any suitable method as will be recognized by one skilled in the art.

Accordingly this invention provides a process for preparing a compound of formula I 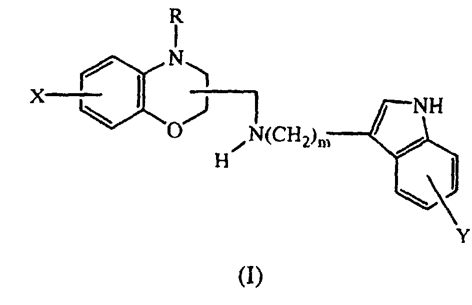 wherein:
R is hydrogen, alkyl of 1 to 6 carbon atoms, phenyl, benzyl or substituted phenyl;
X and Y arc each, independently, hydrogen, halogen, cyano, or alkoxy of 1 to 6 carbon atoms; and
m is 1 to 5; or
a pharmaceutically acceptable salt thereof,
which comprises one of the following:
a) reducing a compound of formula II 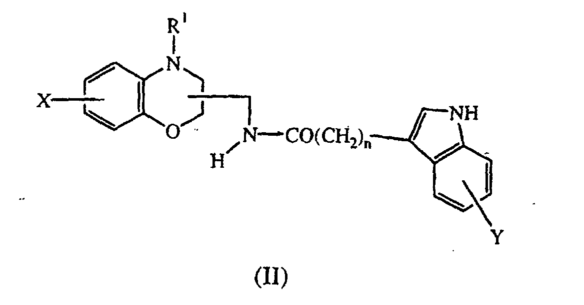 wherein n= 0 to 4, and X and Y are as defined above and R¹ is R as hereinbefore defined or R¹ is COO^{t}Bu to give a compound of formula I wherein m = n +1,
   or
b) reducing a compound of formula III 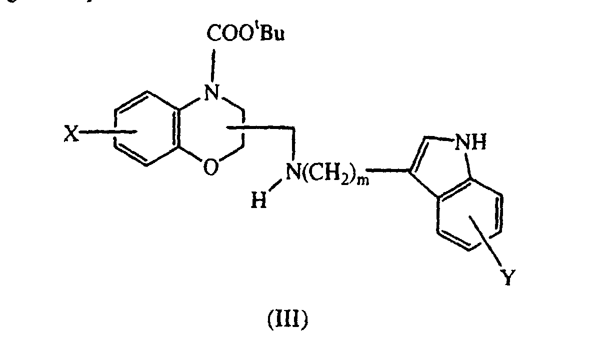 wherein m, X and Y are as defined above to give a corresponding compound of formula I wherein R is methyl;
   or
c) reacting a compound of formula 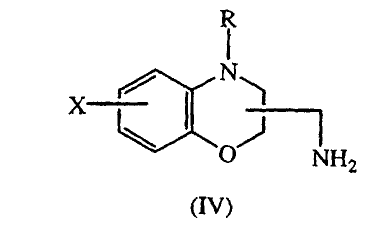 wherein R and X are as hereinbefore defined,
   with a compound of formula 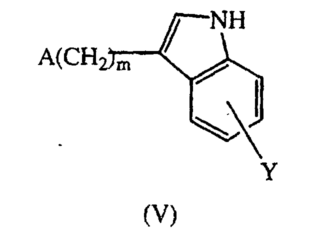 wherein m and Y are as hereinbefore defined and A is a leaving group selected from halogen or an organic sulphonyloxy group e.g aryl or alkylsulphonyloxy group such as tosyloxy;
   or
d) reacting a compound of formula 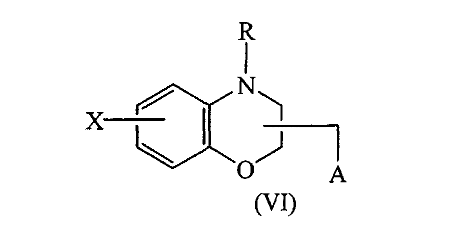 wherein X and R are as defined above and A is a leaving group as defined above,
   with a compound of formula 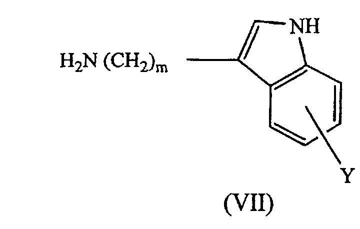 wherein m and Y are as hereinbefore defined;.
   or
e) deprotecting a compound of formula 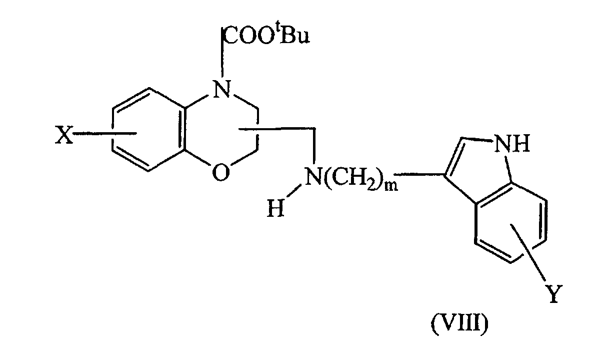 wherein m, X and Y are as defined above to give a compound of formula I wherein is R hydrogen;
   or
f) resolving a mixture (e.g racemate) of optically active isomers of a compound of formula I to isolate one enantiomer or diastereomer substantially free of the other enantiomer or diastereomers;
   or
g) acidifying a basic compound of formula I with a pharmaceutically acceptable acid to give a pharmaceutically acceptable salt.

With regard to process a) above the reduction may be carried out using a reducing agent such as lithuim aluminium hydride suitable for reducing an amide to an amine. Where the group R¹ is CO₂^{t}Bu then that may also be reduced to give a final product of formula I wherein R is methyl.

With regard to process b) above the compounds of formula III may be reduced in similar manner to give a compound of formula I wherein R is methyl.

Processes c) and d) above may be carried out under basic conditions e) in the presence of triethylamine with heating if required

Examples of leaving groups are halogen radicals e.g Br. If necessary reactive groups e.g when R is hydrogen, may be protected by prior to reaction and the protecting group removed thereafter.

Removal of a protecting group is illustrated in process e) above where the BOC protecting group is removed e.g. using trifluoroacetic acid as shown hereinafter in Examples 16 and 17.

The compounds of formula I can possess at least one asymmetric centre and accordingly the compounds may exist and be isolated in a number of optically active stereoisomeric forms. This invention encompasses the compounds of formula I in any optically active form or mixtures thereof eg, racemates. Standard separation techniques may be used to isolate particular enantiomeric or diastereomeric forms. For example a racemic mixture may be converted to a mixture of optically active diastereoisomers by reaction with a single enantiomer of a 'resolving agent' (for example by diastereomeric salt formation or formation of a covalent bond). The resulting mixture of optically active diastereoisomers may be separated by standard techniques (e.g crystallisation or chromatography) and individual optically active diastereoisomers then treated to remove the 'resolving agent' thereby releasing the single enantiomer of the compound of the invention. Chiral chromatography (using a chiral support, eluent or ion pairing agent) may also be used to separate enantiomeric mixtures directly.

The compounds of formula I may be isolated in the form of a salt of a pharmaceutically acceptable acid, e.g. an organic or inorganic acid by treatment with an acid such as described above.

The starting materials/reactants used in the processes above are known or can be made by methods known in the art from readily available materials by processes known or readily apparent to those skilled in the art

However, the present compounds may be advantageously prepared according to any one of Schemes 1, 2, 3, or 4 set forth below: 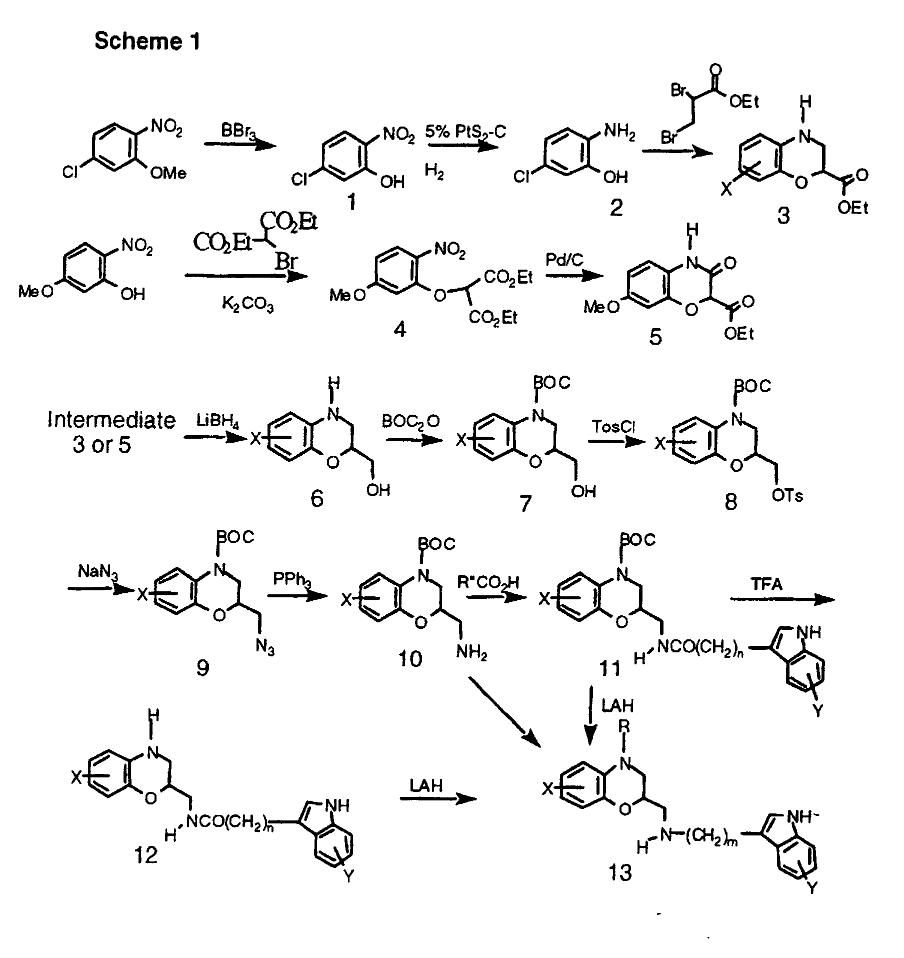 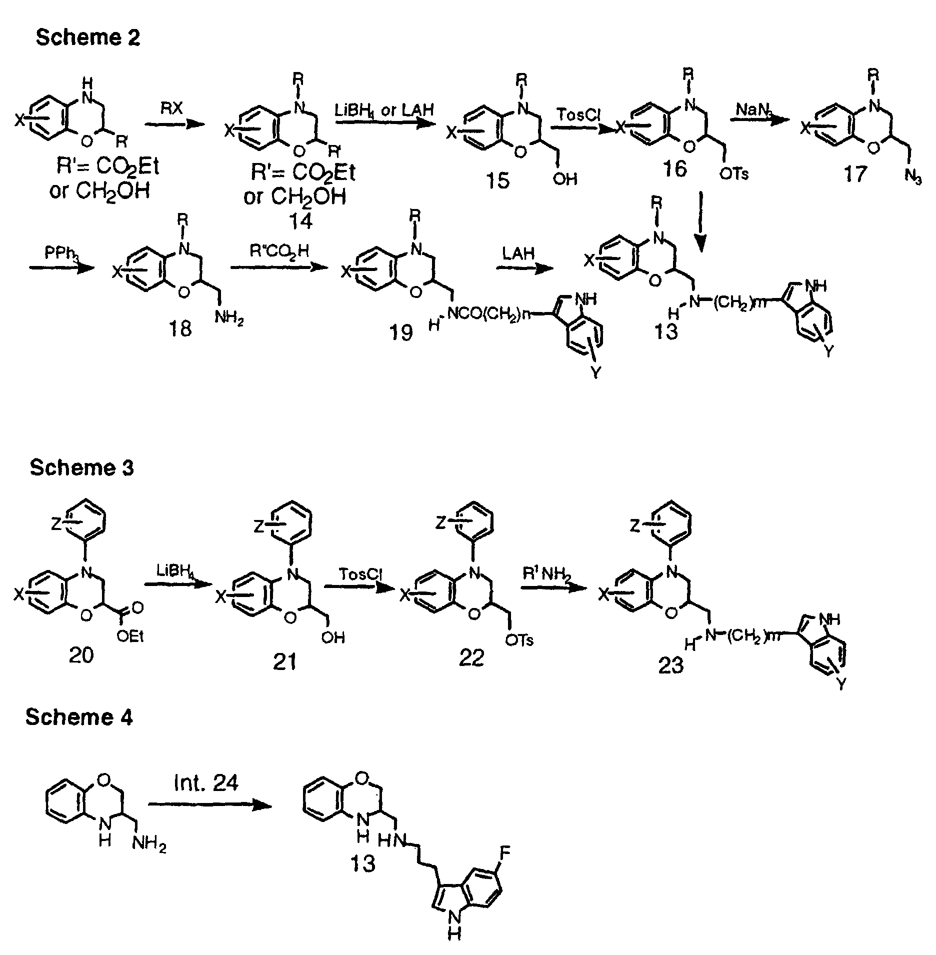 In Schemes 1 to 4, examples are:
R may be methyl, ethyl, propyl, isopropyl or isobutyl;
R' may be CO₂Et or CH₂OH;
R" may be 3-indolyl-propyl or 3-indolyl-butyl;
X may be hydrogen, chlorine or methoxy;
Y may be hydrogen or fluorine;
Z may be hydrogen;
n may be 2 or 3; and
m may be 3 or 4.

Specific exemplification of the production of representative compounds of this invention is given in the following procedures.

### INTERMEDIATE 1

### 2-Nitro-5-chloro-phenol

To a solution of 5-chloro-2-nitroanisole (10 g, 53 mmol) in methylene chloride (30 mL) was added 1 M boron tribromide (11 mL, 0.11 mol) at -78 °C. The reaction mixture was stirred for 3 hours and allowed to warm to room temperature while stirring for another 12 hours. The reaction was quenched by the cautious addition of water (50 mL) and ether (200 mL) was added to the reaction mixture. The mixture was filtered through celite and the organic phase separated and dried over anhydrous sodium sulfate, filtered, and the solvent removed under vacuum. Chromatography (ethyl acetate-hexanes: 1-4) afforded 8.9 g (96 %) of a yellow solid: 31-33 °C.

| Elemental analysis calculated for C₆H₄N₃ClO | | | |
|---|---|---|---|
| Theory | C, 41.52 | H, 2.32 | N, 8.07 |
| Found | C, 41.16 | H, 2.18 | N, 8.02 |

### INTERMEDIATE 2

### 2-Amino-5-chloro-phenol

To a solution of 2-nitro-5-chloro-phenol (2.2 g, 14.0 mmol) in ethanol (40 mL) was added PtS₂-C (5%, 200 mg) and hydrogenated at 45 psi for 4 hours. The mixture was filtered through celite and washed with ethyl acetate (100 mL). The solvent was evaporated and purified by chromatography ((ethyl acetate-hexanes: 2-3) to afford 1.89 g (95 %) of a light brown solid. Recrystallization afforded an analytical sample: mp 149-151 °C.

| Elemental analysis calculated for C₆H₆N₃ClO | | | |
|---|---|---|---|
| Theory | C, 50.20 | H, 4.21 | N, 9.78 |
| Found | C, 49.88 | H, 4.20 | N, 10.06 |

### INTERMEDIATE 3

**Ethyl 7-chloro-2,3-dihydro-benzo[1,4]oxazine-2-carboxylate ester** To a solution of 2-amino-5-chloro-phenol ((1.89 g, 13.2 mmol) in acetone (40 mL) was added potassium carbonate (0.6 g, 4.4 mmol) followed by ethyl 1,2-dibromo-propionic ester (1.14 g, 24.4 mmol) at reflux temperature. After 20 minutes another 0.6 g of potassium carbonate, and 0.64 mL of by ethyl 1,2-dibromo-propionic ester were added, followed by a similar addition of reagents after another 1 hour. The reaction was heated to reflux for 18 hours and the precipitates filtered off and the solvent removed under vacuum. The residue was dissolved in cold IN sodium hydroxide (100 mL) and extracted with ether (3x100 mL). The combined organic layers were washed with water (100 mL) and dried over anhydrous sodium sulfate, filtered, and the solvent removed under vacuum. Chromatography (ethyl acetate hexanes: 1-2) afforded 2.3 g (72 %) of a red solid: mp 82-84 °C.

| Elemental analysis calculated for C₁₁H₁₂NClO₃ | | | |
|---|---|---|---|
| Theory | C, 54.67 | H, 5.00 | N, 5.79 |
| Found | C, 54.27 | H, 4.82 | N, 5.71 |

### INTERMEDIATE 4

**(4-Methoxy-2-nitro-phenoxy)-malonic acid diethyl ester** A mixture of 5-methoxy-2-nitro phenol ( 2.0 g, 12.2 mmol), diethyl bromo malonate (3.5 g, 14.7 mmol), and potassium carbonate (3.4 g, 24.5 mmol) in 2-butanone (70 mL) was heated to reflux for 18 hours. The inorganic salts were filtered and the solvent removed under vacuum. Chromatography (ethyl acetate-hexanes, 1-1) afforded 3.5 g (87 %) of product as a yellow oil: MS (EI) *m*/*e* 327 (M+).

### INTERMEDIATE 5

### 7-Methoxy-3-oxo-3,4-dihydro-2H-benzo[1,4]oxazine-2-carboxylic acid ethyl ester

A mixture of (4-methoxy-2-nitro-phenoxy)-malonic acid diethyl ester (2.3 g, 7.0 mmol) in ethanol containing 10 % Pd/C was hydrogenated at 35 psi for 18 hours. The catalyst was filtered through celite and the solvents were removed under vacuum to afford a solid which was washed with ether to afford 1.25 g of a white solid. The mother liquor was concentrated and the solid again washed with ether to afford another 100 mg of product. Yield: (1.35g, 76 %): mp 138.5-139.5 °C.

| Elemental analysis calculated for C₁₂H₁₃NO₅ | | | |
|---|---|---|---|
| Theory | C, 57.37 | H, 5.22 | N, 5.58 |
| Found | C, 57.36 | H, 5.08 | N, 5.80 |

### INTERMEDIATE 6

### (3,4-Dihydro-2H-benzo[1,4]oxazin-2-yl)-methanol

To a solution of ethyl 2,3-dihydro-benzo[1,4]oxazine-2-carboxylate ester (11.9 g, 19.0 mmol) in anhydrous tetrahydrofuran (60 mL) was added a 2 M solution of lithium borohydride (15 mL) at room temperature. The reaction was stirred for 1 hour and then quenched by the slow addition of methanol. After 2 hours water was slowly added (100 mL) and the reaction mixture was extracted with ethyl acetate (4 x 100 mL). The organic layer was separated and dried over anhydrous magnesium sulfate, filtered, and the solvent removed under vacuum. Purification by chromatography (ethyl acetate-hexane-methanol-3:6:1) to afford 1.96 g (62 %) of an oil: MS (EI) *m*/*e* 165 (M+).

6b) **(7-Methoxy-3,4-dihydro-2H-benzo[1,4]oxazin-2-yl)-methanol** was prepared in a similar fashion using7-methoxy-3-oxo-3,4-dihydro-2H-benzo[1,4]-oxazine-2-carboxylic acid ethyl ester (2.0 g, 8.0 mmol) in 49 % yield (760 mg) as a brown oil: MS (EI) *m*/*e* 196 (M+).

### INTERMEDIATE 7

### 2-Hydroxymethyl-2,3-dihydro-benzo[1,4]oxazine-4-carboxylic acid tert-butyl ester

To a solution of (3,4-dihydro-2H-benzo[1,4]oxazin-2-yl)-methanol (10.7 g, 65.0 mmol) prepared above in anhydrous tetrahydrofuran (200 mL) was slowly added di-tert-butyl dicarbonate (62 g) in tetrahydrofuran (40 mL). The reaction was heated to reflux for 4 hours and allowed to cool to room temperature and then poured into water (100 mL) and extracted with ethyl ether (3 x 100 mL). The organic layer was washed with water (2 x 50 mL) and the organic layer dried over anhydrous sodium sulfate, filtered, and the solvent removed under vacuum. Chromatography (ethyl acetate-hexanes: 1:2) afforded 12.9 g of white solid (75 %): mp 93.5-94.5 °C; MS (EI) *m*/*e* 265 (M+).

| Elemental analysis calculated for C₁₄H₁₉NO₄ | | | |
|---|---|---|---|
| Theory | C, 63.38 | H, 7.22 | N, 5.28 |
| Found | C, 63.53 | H, 7.32 | N, 5.38 |

7b) **2-Hydroxymethyl-7-methoxy-2,3-dihydro-benzo[1,4]oxazine-4-carboxylic acid tert-butyl ester** was prepared from (7-methoxy-3,4-dihydro-2H-benzo[1,4]-oxazin-2-yl)-methanol (1.75 g, 7.0 mmol) as described above in 86 % yield (470 mg) as a clear oil: MS (El) *m*/*e* 295 (M+).

### INTERMEDIATE 8

### 2-(Toluene-4-sulfonyloxymethyl)-2,3-dihydro-benzo[1,4]oxazine-4-carboxylic acid tert-butyl ester

To a solution of 2-hydroxymethyl-2,3-dihydro-benzo[1,4]oxazine-4-carboxylic acid tert-butyl ester (2.8 g, 10.6 mmol) and p-toluenesulfonyl chloride (3.4 g,) in anhydrous pyridine (45 mL) was allowed to stir overnight at room temperature. The reaction mixture was quenched with 1 N sodium hydroxide (50 mL) and extracted with methylene chloride (5 x 50 mL). The organic layer was washed with water (3 x 50 mL) and the organic layer dried over anhydrous sodium sulfate, filtered and the solvent removed under vacuum. Chromatography (ethyl acetate-hexane, 1-3) afforded a thick oil: MS (FAB) *m*/*e* 419 (M+Na).

| Elemental analysis calculated for C₂₁H₂₅NO₆S | | | |
|---|---|---|---|
| Theory | C, 60.13 | H, 6.01 | N, 3.34 |
| Found | C, 60.13 | H, 6.11 | N, 3.56 |

8b) **2-(Toluene-4-sulfonyloxymethyl)-7-methoxy-2,3-dihydro-benzo [1,4]oxazine-4-carboxylic acid tert-butyl ester** (1.4 g, 3.1 mmol) was prepared from 2-hydroxymethyl-7-methoxy-2,3-dihydro-benzo[1,4]oxazine-4-carboxylic acid tert-butyl ester (1.1 g, 3.7 mmol) as described above in 83 % yield as a thick yellow oil: MS (EI) *m*/*e* 449 (M+).

### INTERMEDIATE 9

### t-Butyl-2,3-dihydro-benzo[1,4]oxazine-4-carboxylate-2-methylazide

A solution of t-butyl-2,3-dihydro-benzo[1,4]oxazine-4-carboxylate-2-methyltosylate (14.2 g, 33.9 mmol) and sodium azide (4.4 g, 67.7 mmol) in anhydrous dimethylformamide (150 mL) was heated to 60 °C for 20 hours. The reaction mixture was poured into water (150 mL) and extracted with methylene chloride (3x150 mL). The combined organic layers were dried over anhydrous sodium sulfate, filtered and the solvent removed under vacuum. Purification by chromatography (hexanes) afforded 8.7 g (88 %) of a white solid: mp 82-83 °C.

| Elemental analysis calculated for C₁₄H₁₈N₄O₃ | | | |
|---|---|---|---|
| Theory | C, 57.92 | H, 6.25 | N, 19.30 |
| Found | C, 58.07 | H, 6.21 | N, 19.03 |

### INTERMEDIATE 10

### t.Butyl-2,3-dihydro-benzo[1,4]oxazine-4-carboxylate-2-methylamine

To a solution of t-butyl-2,3-dihydro-benzo[1,4]oxazine-4-carboxylate-2-methylazide (6.25 g, 21.6 mmol) and triphenylphosphine (6.4 g) in tetrahydrofuran (150 mL) containing water (4 mL) was allowed to stir at room temperature for 18 hours. The solvent was removed under vacuum. The residue was dissolved in ethyl ether (100 mL), followed by the addition of hexanes (50 mL). The precipitated triphenylphosphine oxide was filtered off and the solvent removed. Purification by chromatography (5% methanol-methylene chloride) afforded 7.2 g of product (which contained a small amount of triphenylphosphine oxide).: MS (FAB) *m*/*e* 265 (M+H)⁺.

### INTERMEDIATE 11

### N-(3,4-dihydro-2H-benzo[1,4]oxazin-2-ylmethyl)-3-(1H-indol-3-yl)-propionamide-4-carboxylic add tert-butyl ester

A solution of 3-dimethylamino-propyl-3-ethyl carbodiimide hydrochloride (4.4 g, 22.8 mmol) and 3-indole propionic acid (4.3 g, 22.7 mmol) in methylene chloride (100 mL) was stirred at room temperature. After 0.5 hours a solution of t-butyl-2,3-dihydro-benzo[1,4]oxazine-4-carboxylate-2-methylamine (3.0 g, 11.4 mmol) in methylene chloride (5 mL) was added and allowed to stir for 18 hours. The reaction mixture was poured into water (50 mL) and extracted with methylene chloride (3x 100 mL). The organic layer was washed with water (2 x 100 mL), followed by 1 N sodium hydroxide (2 x 50 mL) and dried over anhydrous sodium sulfate, filtered, and the solvent removed under vacuum. Initial chromatography using 5 % methanolmethylenc chloride allowed removal of the triphenylphosphine oxide. Another chromatography (methylene chloride-acetone: 4;1) afforded 3.7 g (61 %) of product as a white foam: MS (FAB) *m*/*z* 436 (M+H)⁺

| Elemental analysis calculated for C₂₅H₂₉N₃O₃ | | | |
|---|---|---|---|
| Theory | C, 68.95 | H, 6.71 | N, 9.65 |
| Found | C, 68.12 | H, 6.72 | N, 9.28 |

### INTERMEDIATE 12

### N-(3,4-Dihydro-2H-benzo[1,4]oxazin-2-ylmethyl)-3-(1H-indol-3-yl)-propionamide

To a solution of N-(3,4-dihydro-2H-benzo[1,4]oxazin-2-ylmethyl)-3-(1H-indol-3-yl)-propionamide-4-carboxylic acid tert-butyl ester (2.2 g, 5.1 mmol) in methylene chloride (100 mL) was added trifluoroacetic acid (20 mL) in methylene chloride (20 mL). The reaction mixture was stirred at room temperature for 2 hours. The reaction mixture was poured into saturated aqueous sodium sulfate (100 mL) and extracted with methylene chloride (2 x 30 mL). The organic layer was again washed with saturated sodium sulfate, followed by water (100 mL). The organic layer was dried over anhydrous magnesium sulfate, filtered, and the solvent removed under vacuum. Purification by chromatography (5 % methanol-methylene chloride) afforded 1.4 g (83 %) of product as a white solid: mp 145-146 °C; MS (FAB) *m*/*z* 336 (M+H)⁺

### INTERMEDIATE 13

### {[3-(5-Fluoro-1H-indol-3-yl)-propylamino]-methyl}-3,4-dihydro-benzo[1,4]oxazine-4-carboxylic acid tert-butyl ester

A solution of t-butyl-2,3-dihydro-benzo[1,4]oxazine-4-carboxylate-2-methylamine (0.9 g, 3.4 mmol), 5-fluoro-indolyl-3-propylbromide [0.58 g, 2.3 mmol; prepared from 3-(5-fluoro-1*H*-indol-3-yl)-propan-1-ol, in the manner described in *J. Med. Chem*. 1976, *19*, 391-395. using CBr₄ and triphenylphosphine], triethylamine (0.345 g, 3.4 mmol) in dimethylsulfoxide (20 mL) was heated to 90 °C for 12 hours. The reaction was quenched with water (100 mL) and extracted with methylene chloride (3x100 mL). The combined organic layers were dried over anhydrous sodium sulfate, filtered, and the solvent removed under vacuum. Chromatography (5 % methanol-methylene chloride) afforded 0.49 g (49 %) of a yellow oil: MS (EI) *m*/*e* 439 (M⁺).

### 13b) {[3-(5-Fluoro-1H-indol-3-yl)-propylamino]-methyl}-7-methoxy-3,4

**dihydro-benzo[1,4]oxazine-4-carboxylic acid tert-butyl ester** (300 mg, 0.64 mmol) was prepared under the condition described above by using t-butyl-7-methoxy-2,3-dihydro-benzo[1,4]oxazine-4-carboxylate-2-methyltosylate (416 mg, 0.92 mmol) and treating with 5-fluoro-indolyl -3-propylamine (0.356 g, 1.85 mmol) in 70 % yield as a yellow glass: MS (EI) *m*/*e* 469 (M+).

### INTERMEDIATE 14 (Method a)

### (4-Benzyl-3,4-dihydro-2H-benzo[1,4]oxazin-2-yl)-methanol

To a solution of (3,4-dihydro-2H-benzo[1,4]oxazin-2-yl)-methanol (4.0 g, 24.0 mmol) in anhydrous acetonitrile (60 mL) was added benzyl bromide (8.6 mL) and stirred at room temperature for 2 hours. The reaction was quenched with water (50 mL) and extracted with methylene chloride (3 x 100 mL). The combined organic layers were dried over anhydrous sodium sulfate, filtered, and the solvent evaporated under vacuum. Chromatography (2.5 % methanol-methylene chloride) afforded 3.7 g (60 %) of product as a yellow oil: MS (FAB) *m*/*e* 255 (M+).

14b) **Ethyl 4-propyl-2,3-dihydro-benzo[1,4]oxazine-2-carboxylate ester** was prepared as a yellow oil in 28 % yield by reacting ethyl 2,3-dihydro-benzo[1,4]oxazine-2-carboxylate ester ( 3.6 g, 17 mmol) and 1-iodopropane (8.6 g, 5.0 mmol) as described above.

| Elemental analysis calculated for C₁₄H₁₉NO₃ | | | |
|---|---|---|---|
| Theory | C, 67.45 | H, 7.68 | N, 5.62 |
| Found | C, 67.79 | H, 7.71 | N, 5.51 |

14c) **Ethyl 7-chloro-4-methyl-2,3-dihydro-benzo[1,4]oxazine-2-carboxylate ester** was prepared by treatment of ethyl 7-chloro-2,3-dihydro-benzo[1,4]oxazine-2-carboxylate ester (2.2g, 9.2 mmol) with methyl iodide (3.9 g, 28 mmol) according to the above procedure in 50 % yield (1.18 g) as a brown oil: MS (EI) *m*/*e* 255 (M⁺).

14d) **(4-Isopropyl-3,4-dihydro-2H-benzo[1,4]oxazin-2-yl)-methanol** In a similar fashion the title compound was prepared by replacing benzyl bromide with isopropyl bromide in 28 % yield (1.6 g) as a yellow oil: MS (El) *m*/*e* 207 (M⁺).

| Elemental analysis calculated for C₁₂H₁₇NO₂ | | | |
|---|---|---|---|
| Theory | C, 69.80 | H, 8.27 | N, 6.76 |
| Found | C, 69.10 | H, 8.13 | N, 6.80 |

### INTERMEDIATE 14e (Method b)

### 4-Isobutyryl-3,4-dihydro-2H-benzo[1,4]oxazine-2-carboxylic acid ethyl_ester

To a solution of ethyl 2,3-dihydro-benzo[1,4]oxazine-2-carboxylate ester (3.0 g, 14.5 mmol), isobutyric acid anhydride (11.4 g, 72 mmol) in tetrahydrofuran (40 mL) was heated to reflux for 18 hours. The reaction was poured into aqueous saturated sodium bicarbonate (50 mL) and extracted with methylene chloride (3x100 mL). The organic layer was dried over anhydrous sodium sulfate, filtered, and the solvent removed under vacuum. Chromatography (ethyl acetate-hexanes: 1-4) afforded 3.4 g (85 %) of product as a yellow oil: MS (EI) *m*/*e* 277 (M⁺).

| Elemental analysis calculated for C₁₅H₁₇₁₉NO₄ | | | |
|---|---|---|---|
| Theory | C, 64.97 | H, 6.91 | N, 5.05 |
| Found | C, 64.32 | H, 7.00 | N, 4.81 |

14f) **4-Acetyl-3,4-dihydro-2H-benzo[1,4]oxazine-2-carboxylic acid ethyl ester** was prepared in 48 % yield as a yellow oil by reacting ethyl 2,3-dihydro-benzo[1,4]oxazine-2-carboxylate ester (3.5 g, 17 mmol) and acetic anhydride (11.3 g, 85 mmol) as described above in method b: MS (EI) *m*/*e* 249 (M⁺).

### INTERMEDIATE 15

### (4-Isobutyl-3,4-dihydro-2H-benzo[1,4]oxazin-2-yl)-methanol

To a solution of 4-isobutyryl-3,4-dihydro-2H-benzo[1,4]oxazine-2-carboxylic acid ethyl ester (3.4 g, 12 mmol) in anhydrous tetrahydrofuran (50 mL) at room temperature was added lithium aluminum hydride (61 mL of 1M solution in tetrahydrofuran, 61 mmol). The reaction was allowed to heat at reflux for 18 hours and the reaction was cautiously quenched by sequentially adding water (4 mL), 15 % NaOH (4 mL), and water (12 mL). The precipitate was filtered and washed with ethyl acetate. The solvent was evaporated under vacuum and the product chromatographed (ethyl acetate-hexanes; 2:3) to afford 1.1 g (25 %) of product as a yellow oil: MS (EI) *m*/*e* 221 (M⁺).

15b) **(4-Ethyl-3,4-dihydro-2H-benzo[1,4]oxazin-2-yl)-methanol** was prepared as a yellow oil in 67 % yield by reacting intermediate 4-acetyl-3,4-dihydro-2H-benzo[1,4]oxazine-2-carboxylic acid ethyl ester as described above: MS (El) *m*/*e* 193 (M⁺).

15c) **(4-Propyl-3,4-dihydro-2H-benzo[1,4]oxazin-2-yl)-methanol** was prepared as a yellow oil as described above in 78 % yield (0.75 g) by using ethyl 4-propyl-2,3-dihydro-benzo[ 1,4]oxazine-2-carboxylate ester (1.15 g, 4.6 mmol).

| Elemental analysis calculated for C₁₂H₁₇NO₂ | | | |
|---|---|---|---|
| Theory | C, 69.54 | H, 8.27 | N, 6.76 |
| Found | C, 69.21 | H, 8.07 | N, 6.81 |

15d) **(7-Chloro-4-methyl-3,4-dihydro-2H-benzo[1,4]oxazin-2-yl)-methanol** was prepared as a yellow oil as described above in 98 % yield (0.91 g) by using ethyl 7-chloro-4-methyl-2,3-dihydro-benzo[1,4]oxazine-2-carboxylate ester (1.1 g, 4.3 mmol): MS (EI) *m*/*e* 213 (M⁺)

### INTERMEDIATE 16

### Toluene sulfonic acid 4-benzyl-3,4-dihydro-2H-benzo[1,4]oxazin-2-yl-methy ester

A solution of (4-benzyl-3,4-dihydro-2H-benzo[1,4]oxazin-2-yl)-methanol (3.9 g, 15.4 mmol) and tosyl chloride (4.2 g) in anhydrous pyridine (80 mL) was stirred for 18 hours and then poured into 1 N HCl (200 mL). The mixture was extracted with methylene chloride (3x 100 mL) and the combined organic layers were washed with 1 N HCl (50 mL), followed by brine (100 mL), then dried over anhydrous sodium sulfate, filtered, and the solvent removed under vacuum. Chromatography (ethyl acetate-hexanes: 1:4) afforded 6.2 g (98 %) of a light yellow oil: MS (FAB) *m*/*z* 409 (M+H)⁺

16b) **Toluene sulfonic acid 4-isopropyl-3,4-dihydro.2H-benzo[1,4]oxazin-2-ylmethy ester** was prepared in a similar fashion using (4-isopropyl-3,4-dihydro-2H-benzo[1,4]oxazin-2-yl)-methanol in 89 % yield as a yellow oil: MS (EI) *m*/*e* 361 (M⁺).

| Elemental analysis calculated for C₁₉H₂₃NO₄S | | | |
|---|---|---|---|
| Theory | C, 63.14 | H, 6.41 | N, 3.80 |
| Found | C, 62.33 | H, 6.22 | N, 3.70 |

16c) **Toluene sulfonic acid 4-isobutyl-3,4-dihydro-2H-benzo[1,4]oxazin-2-ylmethyl ester** was prepared as above using (4-isobutyl-3,4-dihydro-2H-benzo[1,4]oxazin-2-yl)-methanol in 59 % yield as a yellow oil.

| Elemental analysis calculated for C₂₀H₂₅NO₄S | | | |
|---|---|---|---|
| Theory | C, 63.98 | H, 6.71 | N, 3.73 |
| Found | C, 63.71 | H, 6.58 | N, 3.55 |

### INTERMEDIATE 17

### (4-Benzyl-3,4-dihydro-2H-benzo[1,4]oxazin-2-yl)-methylazide

A solution of toluene sulfonic acid 4-benzyl-3,4-dihydro-2H-benzo[1,4]oxazin-2-yl-methyl ester (7.2 g, 17.6 mmol) and sodium azide (2.3 g, 34 mmol) in anhydrous dimethylformamide (40 mL) was heated to 60 °C for 1 day. The mixture was poured into water (200 mL) and extracted with methylene chloride (3x100 mL). The combined organic layers were washed with water (50 mL) and dried over anhydrous sodium sulfate, filtered, and the solvent removed under vacuum. Chromatography (ethyl acetate-hexanes: 1-5) provided 3.6 g (72 %) of a yellow oil.

| Elemental analysis calculated for C₁₆H₁₆N₄O₁ | | | |
|---|---|---|---|
| Theory | C, 68.55 | H, 5,75 | N, 19.99 |
| Found | C, 68.38 | H, 5.56 | N, 19.85 |

### INTERMEDIATE 18

### (4-Benzyl-3,4-dihydro-2H-benzo[1,4]oxazin-2-yl)-methylamine

(4-Benzyl-3,4-dihydro-2H-benzo[1,4]oxazin-2-yl)-methylazide (3.5 g, 12 mmol) was reacted according to the procedure used for Intermediate 10 to afford 2.3 g (73 %) of the title compound as a yellow oil. The oxalate salt was prepared in ethanol: mp 214-215 °C.

| Elemental analysis calculated for C₁₆H₁₈N₂O | | | |
|---|---|---|---|
| Theory | C, 62.72 | H, 5.85 | N, 8.13 |
| Found | C, 62.58 | H, 5.76 | N, 8.04 |

### INTERMEDIATE 18b (Method b)

### (4-Methyl-3,4-dihydro-2H-benzo[1,4]oxazin-2-yl)-methylamine

To a solution of t-butyl-2,3-dihydro-benzo[1,4]oxazine-4-carboxylate-2-methylazide (1.9 g, 6.5 mmol) in anhydrous tetrahydrofuran (30 mL) was added 32 mL of IM lithium aluminum hydride in tetrahydrofuran and heated to reflux for 18 hours. The reaction mixture was quenched with a saturated solution of ammonium chloride and the precipitate filtered through celite. The celite was washed with methanol containing 1% aqueous ammonium hydroxide. The solvent was evaporated under vacuum and the product purified by chromatography (10% methanol-methylene chloride) to afford 0.45 g of the title compound as a yellow oil: MS (FAB) *m*/*z* 179 (M+H)⁺.

### INTERMEDIATE 19

### N-(4-Benzyl-3,4-dihydro-2H-benzo[1,4]oxazin-2-ylylmethyl)-3-(1H-indol-3-yl)-propionamide

(4-Benzyl-3,4-dihydro-2H-benzo[1,4]oxazin-2-yl)-methylamine (0.9 g, 3.5 mmol)was coupled to indole propionic acid (1.6 g, 8.2 mmol) according to the procedure used to prepare intermediate 11 above to afford 1.5 g (98 %) of the title compound as a white solid: mp 39-61 °C; MS (EI) *m*/*e* 425 (M⁺).

| Elemental analysis calculated for C₂₇H₂₇N₃O₂ | | | |
|---|---|---|---|
| Theory | C, 76.21 | H, 6.39 | N, 9.87 |
| Found | C, 75.40 | H, 6.53 | N, 9.46 |

19b) **N-(4-Benzyl-3,4-dihydro-2H-benzo[1,4]oxazin-2-ylylmethyl)-3-(lH-indol-3-yl)-butyramide.** (4-Benzyl-3,4-dihydro-2H-benzo[1,4]oxazin-2-yl)-methylamine (1.2 g, 4.7 mmol) was coupled to indole butyric acid (1.8 g, 8.9 mmol) according to the procedure used to prepare intermediate 11 above to afford 1.3 g (63 %) of the title compound as a yellow solid: mp 61-63.5 °C.; MS (EI) *m*/*e* 439 (M⁺)

### INTERMEDIATE 20

### Ethyl 4-phenyl-2,3-dihydro-benzo[1,4]oxazine-2-carboxylate ester

A solution of ethyl 2,3-dihydro-benzo[1,4]oxazine-2-carboxylate ester (10g, 48 mmol), 1,4-cyclohexanedione (10.8 g, 97 mmol) and p-toluenesulfonic acid (2 g) in toluene (200 mL) was heated to reflux for 4 hours. The solvent was evaporated and the product was purified by chromatography (ethyl acetate-hexane: 1-3) to afford 7.2 g (53 %) of product as a yellow oil: MS (EI) *m*/*e* 283 (M⁺).

### INTERMEDIATE 21

### (4-Phenyl-3,4-dihydro-2H-benzo[1,4]oxazin-2-yl)-methanol

To a solution of ethyl 4-phenyl-2,3-dihydro-benzo[1,4]oxazine-2-carboxylate ester (6.9 g, 24 mmol) in anhydrous tetrahydrofuran (80 mL) was added 60 mL of 2 M lithium borohydride in tetrahydrofuran (0.12 mol) at room temperature. The reaction mixture was stirred at room temperture for 4 hours and quenched with methanol. The reaction was poured into water (100 mL) and extracted with ether (3x 80 mL) and the combined organic layers dried over anhydrous sodium sulfate, filtered, and the solvent removed under vacuum. Chromatography (ethyl aceate-hexanes; 1-3) afforded 5.7 g (96 %) of product as a clear oil: MS (EI) *m*/*e* 213 (M⁺).

| Elemental analysis calculated for C₁₅H₁₅NO₂ | | | |
|---|---|---|---|
| Theory | C, 74.67 | H, 6.27 | N, 5.81 |
| Found | C, 74.21 | H, 6.60 | N, 5.56 |

### INTERMEDIATE 22

### Toluene sulfonic acid 4-phenyl-3,4-dihydro-2H-benzo[1,4]oxazin-2-yl-methyl ester

(4-Phenyl-3,4-dihydro-2H-benzo[1,4]oxazin-2-yl)-methanol (5.6 g, 23 mmol) was reacted according to the procedure as described above for Intermediate 8 to afford 8.2 g (89 %) of the title compound: mp 83-85°C.

| Elemental analysis calculated for C₂₁H₂₁NO₄S | | | |
|---|---|---|---|
| Theory: | C, 66.82 | H, 5.35 | N, 3.80 |
| Found: | C, 66.53 | H, 5.39 | N, 3.40 |

22b) **Toluene-4-sulfonic acid 4-ethyl-3,4-dihydro-2H-benzo[1,4]oxazin-2-yl-methyl ester** was prepared as described above using (4-ethyl-3,4-dihydro-2H-benzo[1,4]oxazin-2-yl)-methanol in 32 % yield as a bluish solid: mp 85-87 °C: MS (EI) *m*/*e 347* (M⁺).

22c) **Toluene-4-sulfonic acid 4-propyl-3,4-dihydro-2H-benzo[1,4]oxazin-2-yl-methyl ester** was prepared as described above as a thick oil in 83 % yield by using (4-propyl-3,4-dihydro-2H-benzo[1,4]oxazin-2-yl)-methanol: MS EI *m*/*e* 361 (M⁺).

22d) **Toluene-4-sulfonic acid 7-chloro-4-methyl-3,4-dihydro-2H-benzo[1,4]oxazin-2-yl-methyl ester** was prepared as described above as a blue solid in 87 % yield by using (7-chloro-4-methyl-3,4-dihydro-2H-benzo[1,4]oxazin-2-yl)-methanol: mp 65-67 °C.; MS El *m*/*e* 367 (M⁺).

### INTERMEDIATE 24

### 5-Fluoro-3-(3-p-toluenesulfonyloxypropyl)indole.

To a stirred solution of 3-(5-fluoro-1*H*-indol-3-yl)-propan-1-ol (2.90 g, 15.0 mmol) in pyridine (15.0 mL) was added *p*-toluenesulfonyl chloride (7.1 g, 37.5 mmol) at room temperature. After stirring for 30 minutes at room temperature, the reaction mixture was poured to 200 mL of ice water. The aqueous was extracted with ethyl acetate and the combined organic extracts were washed with IN HCl, brine, dried over anhydrous sodium sulfate, filtered, and the solvent was concentrated. The crude product was purified by silica gel column chromatography (ethyl acetate/hexane, 3/7) to give 4.1 g (79 %) of the title compound as a solid: mp 74 °C (Lit. mp 99 °C; EP 464604 A2).

The following non-limiting examples illustrate the preparation of representative compounds of this invention.

### EXAMPLE 1

### (3,4)-Dihydro-2H-benzo[1,4]oxazin-2-yl-methyl)-[3-(1H-indo-3-yl)-propyl]-amine

To a solution of lithium aluminum hydride (0.78g), in anhydrous tetrahydrofuran (20 mL) was added 1.1 g of N-(3,4-dihydro-2H-benzo[1,4]oxazin-2-ylylmethyl)-3-(1H-indol-3-yl)-propionamide in tetrahydrofuran (20 mL). The reaction mixture was allowed to reflux for 18 hours then cooled to room temperature and quenched with aqueous ammonium chloride. The precipitate was filtered through celite and the solvent removed under vacuum. Purification by chromatography (5% methanol:methylene chloride) provided 0.9 g (90 %) of a white foam: MS (EI) *m*/*e* 321 (M⁺).
The fumarate salt was prepared in ethanol: mp 203-204 °C.

| Elemental analysis calculated for C₂₀H₂₃N₃O₃·C₄H₄O₄ | | | |
|---|---|---|---|
| Theory | C, 68.55; | H, 6.01; | N, 10.90 |
| Found | C, 68.93; | H, 6.47; | N, 10.98 |

### EXAMPLE 2

### 3-(1H-Indol-3-yl)-propyl]-(4-methyl-3,4-dihydro-2H-benzo[1,4]oxazin-2-yl-methyl)-amine

To a solution of lithium aluminum hydride (0.69g) in anhydrous tetrahydrofuran (50 mL) was added {[3-(1H-indol-3-yl)-1-oxo-propylamino]-methyl}-3,4-dihydro-benzo[1,4]oxazine-4-carboxylic acid tert-butyl ester (1.3 g, 3.0 mmol) in tetrahydrofuran (10 mL) at 0°C. The reaction mixture was heated to reflux for 18 hours and then quenched with aqueous ammonium chloride and the solids filtered through celite. The solvent was removed under vacuum and the product was purified by chromatography (5 % methanol-methylene) to provide 0.51 g (51%) as a pale-yellow solid: mp 110-112 °C. The fumarate salt was prepared in ethanol to afford yellow crystals: mp 196.5-197.5 °C.

| Elemental analysis calculated for C₂₁H₂₃N₃O₃·C₄H₄O₄ | | | |
|---|---|---|---|
| Theory | C, 66.5; | H, 6.47; | N, 9.31 |
| Found | C, 66.21; | H, 6.38; | N, 9.18 |

### EXAMPLE 3

### (4-Benzyl-3,4-dihydro-2H-benzo[1,4]oxazin-2ylmethyl)-[3-(1H-indo-3-yl)-propyl]-amine

This compound was prepared from N-(4-benzyl-3,4-dihydro-2H-benzo[1,4]oxazin-2-ylmethyl)-3-(1H-indol-3-yl)-propionamide according to the procedure set forth in Example 2 to provide an 87 % yield. The fumarate salt was prepared in isopropanol: mp 174-176 °C.

| Elemental analysis calculated for C₂₇H₂₉N₃O·C₄H₄O₄ | | | |
|---|---|---|---|
| Theory | C, 70.57; | H, 6.3; | N, 7.96 |
| Found | C, 70.26; | H, 6.23; | N, 7.8 |

### EXAMPLE 4

### (4-Benzyl-3,4-dihydro-2H-benzo[1,4]oxazin-2ylmethyl)-[3-(1H-indo-3-yl)-butyl]-amine

This compound was prepared from N-(4-benzyl-3,4-dihydro-2H-benzo[1,4]oxazin-2-ylmethyl)-3-(1H-indol-3-yl)-butyramide according to the procedure set forth in Example 2 to provide a 73 % yield. The fumarate salt was prepared in ethanol: mp 171.5-172.5 °C.

| Elemental analysis calculated for C₂₈H₃₁N₃O·0.5C₄H₄O₄ | | | |
|---|---|---|---|
| Theory | C, 74.51; | H, 6.88; | N, 8.69 |
| Found | C, 74.12; | H, 6.80; | N, 8.61 |

### EXAMPLE 5

### [3-(5-Fluoro-1H-indol-3-yl)-propyl]-(4-methyl-3,4-dihydro-2H-benzo[1,4]oxazin-2-yl-methyl)-amine

A solution of 4-methyl-3,4-dihydro-2H-benzo[1,4]oxazin-2-yl-methylamine (0.45 g, 2.5 mmol) and 5-fluoro-indolyl -3-propylbromide (0.6 g, 2.5 mmol), and triethylamine (0.5 g) in dimethylsulfoxide (20 mL) was heated to 100 °C for 10 hours. The reaction mixture was poured into water (100 mL) and extracted with methylene chloride (3x 50 mL). The combined organic layers were dried over anhydrous sodium sulfate, filtered, and the solvent removed under vacuum. Chromatography (10% methanol-methylene chloride) afforded 0.42 g (47 %) of desired product as a yellowish brown oil. The oxalate salt was prepared in ethanol: mp 189-190 °C.

| Elemental analysis calculated for C₂₁H₂₁N₃O·C₂H₂O₄ | | | |
|---|---|---|---|
| Theory | C, 62.25; | H, 5.91; | N, 9.47 |
| Found | C, 62.03; | H, 6.06; | N, 9.41 |

### EXAMPLE 6

### [3-(1H-Indol-3-yl)-propyl]-4-benzyl-3,4-dihydro-2H-benzo[1,4]oxazin-2-yl-methyl)-amine

This compound was prepared in 63 % yield as a yellow oil as described in Example 5 by reacting 4-benzyl-3,4-dihydro-2H-benzo[1,4]oxazin-2-yl)-methylamine (0.21 g, 0.83 mmol) with 3-(5-fluoro-3-indolylpropyl)bromide (0.14 g, 5.5 mmol) to provide a 63% yield. The oxalate salt was prepared in ethanol: mp 212-214 °C.

| Elemental analysis calculated for C₂₇H₂₈N₃OF·C₂H₂O₄ | | | |
|---|---|---|---|
| Theory | C, 66.46; | H, 5.87; | N, 8.02 |
| Found | C, 66.29; | H, 5.74; | N, 8.04 |

### EXAMPLE 7

### [3-(1H-Indol-3-yl)-propyl]-4-phenyl-3,(4-dihydro-2H-benzo[1,4]oxazin-2-yl-methyl)-amine

A mixture of toluene sulfonic acid 4-phenyl-3,4-dihydro-2H-benzo[1,4]oxazin-2-yl-methyl ester (1.2 g, 3.0 mmol) and 3-(3-indolyl)propylamine (1.13 g, 6 mmol) in dimethylsulfoxide (20 mL) was heated to 100 °C for 12 hours. The reaction mixture was poured into water (100 mL) and extracted with methylene chloride (3x100 mL). The combined orgainic layers were washed with water (3x100 mL) and dried over anhydrous sodium sulfate, filtered, and the solvent removed under vacuum. Chromatography (5 % methanol-methylene chloride) afforded 0.96 g (80 %) of product as a yellow oil. The fumarate salt was prepared in ethanol: mp 197-198°C.

| Elemental analysis calculated for C₂₆H₂₇N₃O·C₄H₄O₄ | | | |
|---|---|---|---|
| Theory | C, 69.55; | H, 6.13; | N, 8.11 |
| Found | C, 69.61; | H, 5.98; | N, 8.04 |

### EXAMPLE 8

### [3-(5-Fluoro-1H-indol-3-yl)-propyl]-(4-isopropyl-3,4-dihydro-2H-benzo[1,4]oxazin-2yl-methyl)-amine

This compound was prepared in a 40 % yield in the manner described in Example 7 by reacting toluene sulfonic acid 4-isopropyl-3,4-dihydro-2H-benzo[1,4]oxazin-2-yl-methyl ester (1 g, 2.8 mmol) with 3-(5-fluoro-3-indolyl)propylamine (0.8 g, 4.2 mmol). The oxalate salt was prepared in ethanol: mp 204-206 °C.

| Elemental analysis calculated for C₂₃H₂₈N₃OF·C₂H₂O₄ | | | |
|---|---|---|---|
| Theory | C, 63.64; | H, 6.41; | N, 8.91 |
| Found | C, 63.31; | H, 6.47; | N, 9.1 |

### EXAMPLE 9

### [3-(1H-indol-3-yl)-butyl]-4-isopropyl-3,(4-dihydro-2H-benzo [1,4]oxazin-2-yl-methyl)-amine

This compound was prepared in a 52 % yield as a yellow oil in the manner described in Example 7 by reacting toluene sulfonic acid 4-isopropyl-3,4-dihydro-2H-benzo[1,4]oxazin-2-yl-methyl ester(0.75 g, 2.1 mmol) with 3-(3-indolyl)butylamine (0.51 g, 2.7 mmol). The oxalate salt was prepared in ethanol: mp 166.5-168 °C.

| Elemental analysis calculated for C₂₄H₃₁N₃O·C₂H₂O₄ | | | |
|---|---|---|---|
| Theory | C, 66.74; | H, 7.11; | N, 8.98 |
| Found | C, 66.45; | H, 6.91; | N, 8.88 |

### EXAMPLE 10

### [3-(5-Fluoro-1H-indol-3-yl)-propyl]-4-isobutyl-3,4-dihydro-2H-benzo[1,4]oxazin-2-yl-methyl)-amine

This compound was prepared as a yellow solid in a 56 % yield in the manner described in Example 7 by reacting toluene sulfonic acid 4-isobutyl-3,4-dihydro-2H-benzo[1,4]oxazin-2-yl-methyl ester (0.69 g, 1.8 mmol) with 3-(5-fluoro-3-indolyl)propylamine (0.53 g, 2.8 mmol): mp 99-101.5 °C The oxalate salt was prepared in ethanol: mp 214-215 °C.

| Elemental analysis calculated for C₂₄H₃₀N₃OF·C₂H₂O₄·0.5H₂O | | | |
|---|---|---|---|
| Theory | C, 63.14; | H, 6.73; | N, 8.50 |
| Found | C, 63.09; | H, 6.53; | N, 8.63 |

### EXAMPLE 11

### [3-(5-Fluoro-1H-indol-3-yl)-propyl]-(4-phenyl-3,4-dihydro-2H-benzo[1,4]oxazin-2-yl-methyl)-amine

This compound was prepared as a brown solid in 48 % yield in the manner described in Example 7 by reacting toluene sulfonic acid (4-phenyl-3,4-dihydro-2H-benzo[1,4]oxazin-2-yl)-methyl ester (0.8 g, 2.0 mmol) with 3-(5-fluoro-3-indolyl)-propylamine (0.58 g, 3.0 mmol): mp 67-70 °C. The oxalate salt was prepared in ethanol: mp 179-180 °C.

| Elemental analysis calculated for C₂₆H₂₆N₃OF·C₂H₂O₄·0.25 H₂O | | | |
|---|---|---|---|
| Theory | C, 65.93; | H, 5.63; | N, 8.24 |
| Found | C, 66.00; | H, 5.47; | N, 8.17 |

### EXAMPLE 12

### [3-(5-Fluoro-1H-indol-3-yl)-butyl]-(4-phenyl-3,4-dihydro-2H-benzo[1,4]oxazin-2-yl-methyl)-amine

This compound was prepared as a brown solid in 84 % yield in the manner described in Example 7 by reacting toluene sulfonic acid 4-phenyl-3,4-dihydro-2H-benzo[1,4]-oxazin-2-yl-methyl ester (1.0 g, 2.5 mmol) with 3-(3-indolyl)butylamine (0.95 g, 5.1 mmol). The oxalate salt was prepared in ethanol: mp 175-177 °C.

| Elemental analysis calculated for C₂₇H₂₉N₃O·C₂H₂O₄ | | | |
|---|---|---|---|
| Theory | C, 69.40; | H, 6.23; | N, 8.37 |
| Found | C, 69.27; | H, 6.19; | N, 8.33 |

### EXAMPLE 13

### [3-(5-Fluoro-1H-indol-3-yl)-propyl]-(4-ethyl-3,4-dihydro-2H-benzo[1,4]oxazin-2-yl-methyl)-amine

This compound was prepared as a yellow solid in a 47 % yield by treating toluene sulfonic acid 4-ethyl-3,4-dihydro-2H-benzo[1,4]oxazin-2-yl-methyl ester (0.6 g, 1.7 mmol) with 3-(5-fluoro-3-indolyl)propylamine (0.59 g, 2.6 mmol): mp 127-129 °C. The oxalate salt was prepared in ethanol: mp 203-204 °C.

| Elemental analysis calculated for C₂₂H₂₆N₃OF·C₂H₂O₄ | | | |
|---|---|---|---|
| Theory | C, 62.96; | H, 6.16; | N, 9.18 |
| Found | C, 62.81; | H, 6.09; | N, 9.10 |

### EXAMPLE 14

### [3-(5-Fluoro-1H-indol-3-yl)-propyl]-(4-propyl-3,4-dihydro-2H-benzo[1,4]oxazin-2-yl-methyl)-amine

This compound was prepared in a 50 % yield as a yellow oil in the manner described in Example 13 by reacting toluene sulfonic acid 4-propyl-3,4-dihydro-2H-benzo[1,4]oxazin-2-yl)-methyl ester (0.9 g, 2.5 mmol) with 3-(5-fluoro-3-indolyl)propylamine (0.7 g, 3.7 mmol).

| Elemental analysis calculated for C₂₃H₂₈N₃OF | | | |
|---|---|---|---|
| Theory | C, 72.41; | H, 7.40; | N, 11.02 |
| Found | C, 72.20; | H, 7.33; | N, 11.01 |

The oxalate salt was prepared in ethanol: mp 215-216 °C.

| Elemental analysis calculated for C₂₃H₂₈N₃OF·C₂H₂O₄ | | | |
|---|---|---|---|
| Theory | C, 63.64; | H, 6.41; | N, 8.91 |
| Found | C, 63.33; | H, 6.45; | N, 8.88 |

### EXAMPLE 15

### (7-Chloro-4-methyl-3,4-dihydro-2H-benzo[1,4]oxazin-2ylmethyl)-[3-(5-fluoro-1H-indol-3-yl)-propyl]-amine

This compound was prepared in a 63 % yield as a yellow oil in the manner described in Example 13 by reacting toluene sulfonic acid 7-chloro-4-methyl-3,4-dihydro-2H-benzo[1,4]oxazin-2-yl-methyl ester (0.6 g, 1.6 mmol) and with 3-(5-fluoro-3-indolyl)propylamine (0.63 g, 3.3 mmol). The fumarate salt was prepared in ethanol: mp 185-186.5 °C.

| Elemental analysis calculated for C₂₁H₂₃N₃OFCl·C₄H₄O₄ | | | |
|---|---|---|---|
| Theory | C, 58.58; | H, 5.40; | N, 8.34 |
| Found | C, 59.44; | H, 5.38; | N, 8.24 |

### EXAMPLE 16

### (3,4-Dihydro-2H-benzo[1,4]oxazin-2ylmethyl)-[3-(5-fluoro-1H-indol-3-yl)-propyl]-amine

The title compound was prepared in a 19 % yield by reacting {[3-(5-fluoro-lH-indol-3-yl)-propylamino]-methyl}-3,4-dihydro-benzo[1,4]oxazine-4-carboxylic acid tert-butyl ester (0.49 g, 1.1 mmol) with trifluoroacetic acid (20 mL) in methylene chloride (20 mL). The reaction mixture was stirred at room temperature for two hours and then poured into saturated sodium carbonate and extracted with methylene chlloride. The organic layer was dried over anhydrous sodium sulfate, filtered and chromatographed (5% MeOH in methylene chloride) afforded 0.06 g (19%) of product as an oil. The oxalate salt was prepared in ethanol: mp 168-170 °C.

| Elemental analysis calculated for C₂₀H₂₂N₃OF·1.5C₂H₂O₄ | | | |
|---|---|---|---|
| Theory | C, 55.49; | H, 5.05; | N, 8.09 |
| Found | C, 55.46; | H, 5.12; | N, 8.39 |

### EXAMPLE 17

### 7-Methoxy-3,4-dihydro-2H-benzo[1,4]oxazin-2ylmethyl)-[3-(5-Fluoro-1H-indol-3-yl)-propyl]-amine

The title compound (186 mg, 0.50 mmol) was prepared according to the procedure of Example 16 by using {[3-(5-fluoro-1H-indol-3-yl)-propylamino]-methyl}-7-methoxy-3,4-dihydro-benzo[1,4]oxazine-4-carboxylic acid tert-butyl ester (290 mg, 0.62 mmol) in 82 % yield as a clear glass: MS (El) *m*/*e* 369 (M+). The oxalate salt was prepared from ethanol: mp 190-191 °C.

| Elemental analysis calculated for C₂₁H₂₄N₃O₂F·2.0C₂H₂O4·0.25H₂O | | | |
|---|---|---|---|
| Theory | C, 54.20; | H, 5.19; | N, 7.58 |
| Found | C, 53.96; | H, 5.22; | N, 7.41 |

### EXAMPLES 18 and 19

### Resolution of [3-(5-Fluoro-1H-indol-3-yl)-propyl]-4-phenyl-3,4-dihydro-2H-benzo[1,4]oxazin-2-yl-methyl)-amine

Racemic [3-(5-fluoro-1H-indol-3-yl)-propyl]-4-phenyl-3,4-dihydro-2H-benzo[1,4] oxazin-2ylmethyl)-amine was resolved on a chiracel column to afford 160 mg of

**(-)-[3-(5-fluoro-1H-indol-3-yl)-propyl]-4-phenyl-3,4-dihydro-2H-benzo[1,4]oxazin-2-yl-methyl)-amine** as a yellow oil (100 % optical purity). The oxalate salt was prepared in ethanol: mp 198-200 °C; [α]_{D} -16.7 (c = DMSO, 10.04 mg/ml). DMSO, 10.04 mg/ml

| Elemental analysis calculated for C₂₆H₂₆N₃OF·C₂H₂O₄ | | | |
|---|---|---|---|
| Theory | C, 66.48; | H, 5.58; | N, 8.31 |
| Found | C, 66.29; | H, 5.57; | N, 8.19 |

followed by **(+)-[3-(5-fluoro-1H-indol-3-yl)-propyl]-4-phenyl-3, 4-dihydro-2H-benzo[1,4]oxazin-2-yl-methyl)-amine** (140 mg,) as a yellow oil (99 % optical purity). The oxalate salt was prepared in ethanol: (WAY 143399); mp 196-197.5 °C; [α]_{D} 18.3 ° (c = DMSO, 9.83 mg/ml). DMSO, 9.83 mg/ml

| Elemental analysis calculated for C₂₆H₂₆N₃OF·C₂H₂O₄·0.25 H₂O | | | |
|---|---|---|---|
| Theory | C, 65.93; | H, 5.63; | N, 8.24 |
| Found | C, 65.99; | H, 5.53; | N, 8.17 |

### EXAMPLE 20

### (3,4-Dihydro-2H-benzo[1,4]oxazin-3-yl-methyl)-[3-(5-fluoro-1H-indol-3-yl)propyl]-amine

To a solution of 5-fluoro-3-(3-*p*-toluenesulfonyloxypropyl)indole (0.52 g, 1.5 mmol) and 3-aminomethyl-1,4-benzoxazine (0.25 g, 1.5 mmol) [prepared in accordance with the procedure set forth in Ind. J. Chem., 13:462-467 (1975)] in acetonitrile (15 mL) was added triethylamine (0.42 mL, 3.0 mmol) at room temperature. The reaction mixture was allowed to reflux 18 h, cooled and concentrated. The crude product was purified by silica gel column chromatography (ethyl acetate/methanol/NH₄OH, 98/2/0.5) to afford 265 mg (52 %) of the free base.

The fumarate salt was prepared in ethanol: mp 185-187 °C: MS (El) *m*/*e* 339 (M⁺).

| Elemental analysis calculated for C₂₀H₂₂FN₃O·0.5C₄H₄O₄0.25H₂O | | | |
|---|---|---|---|
| Theory | C, 65.74; | H, 6.14; | N, 10.45 |
| Found | C, 65.71; | H, 6.16; | N, 10.22 |

The compounds of the present invention have a high affinity for the 5-HT_{1A} receptor and an ability to inhibit 5-HT transport. Therefore, the present compounds are useful in the treatment of CNS disorders amenable to treatment with antidepressant and anxiolytic agents. The activity of the present compounds is demonstrated by the following standard pharmacological test procedure.

The PCR cloning of the human 5-HT_{1A} receptor subtype from a human genomic library has been described previously in Chanda et al., Mol. Pharmacol., 43:516 (1993). A stable Chinese hamster ovary cell line expressing the human 5-HT_{1A} receptor subtype (5H-HT_{1A}.CHO cells) was employed throughout this procedure. Cells were maintained in DMEM supplemented with 10% fetal calf serum, non-essential amino acids and penicillin/ streptomycin.

Cells were grown to 95-100% confluency as a monolayer before membranes were harvested for binding studies. Cells were gently scraped from the culture plates, transferred to centrifuge tubes, and washed twice by centrifugation (at 2000 rpm for 10 min., 4°C) in buffer (50 mM Tris; pH 7.5). The resulting pellets were aliquoted and stored at -80•C until needed. On the day of assay, the cells were thawed on ice, and resuspended in buffer. Studies were conducted using [³H]8-OH-DPAT as the radioligand. The binding assay was performed in 96 well microtiter plates in a final total volume of 250 µL of buffer. Competition experiments were performed by using 7 concentrations of unlabelled drug and a final ligand concentration of 1.5 nM . Non-specific binding was determined in the presence of 10 µM 5HT. Saturation analysis was conducted by using [³H]8-OH-DPAT at concentrations ranging from 0.3-30 nM. Following a 30 minute incubation at room temperature, the reaction was terminated by the addition of ice cold buffer and rapid filtration using a M-96 Brandel Cell Harvester through a GF/B filter presoaked for 30 minutes in 0.5% polyethyleneimine.

A protocol similar to that used by Cheetham et al., Neuropharmacol., 32:737, (1993), was used to determine the affinity of compounds for the serotonin transporter. Briefly, frontal cortical membranes prepared from male Sprague-Dawley rats were incubated with ³H-paroxetine (0.1 nM) for 60 min at 25°C. All tubes also contained either vehicle, test compound (one to eight concentrations), or a saturating concentration of fluoxetine (10 µM) to define specific binding. All reactions were terminated by the addition of ice cold Tris buffer followed by rapid filtration using a Tom Tech filtration device to separate bound from free ³H-paroxetine. Bound radioactivity was quantitated using a Wallac 1205 Beta Plates counter. Nonlinear regression analysis was used to determine IC₅₀ values which were converted to Ki values using the method of Cheng and Prusoff, Biochem. Pharmacol., 22: 3099 (1973): Ki = IC50/((Radioligand conc.)/(1 + KD)).

The degree of agonism/antagonism at the 5-HT_{1A} receptor using [³⁵S]-GTPγS binding to cloned human 5-HT_{1A} receptors was determined as follows. The [³⁵S]-GTPγS binding assay was similar to that used by Lazareno and Birdsall, Br. J. Pharmacol., 109: 1120, (1993). Briefly, 5-HT_{1A} cloned receptor membrane fragments (as used for 5-HT_{1A} receptor binding assays) were stored at -70°C until needed. When needed, membranes were rapidly thawed, centrifuged at 40,000 x g for 10 minutes and resuspended at 4°C for 10 minutes in assay buffer (25 mM HEPES, 3 mM MgCl₂, 100 mM NaCI, 1 mM EDTA, 10 uM GDP, 500 mM DTT, pH 8.0). These membranes were then incubated for 30 min at 30 °C with [³⁵S]GTPgS (1 nM) in the presence of vehicle, test compound (one to eight concentrations), or excess 8-OH-DPAT to define maximum agonist response. All reactions were terminated by the addition of ice cold Tris buffer followed by rapid filtration using a Tom Tech® filtration device to separate bound from free [³⁵S]GTPgS. Agonists produce an increase in the amount of [³⁵S]GTPgS bound whereas antagonists produce no increase in binding. Bound radioactivity was counted and analyzed as above.

The following assays were performed by incubating the cells with DMEM containing 25 mM HEPES, 5 mM theophylline and 10 µM pargyline for a period of 20 minutes at 37°C. Functional activity was assessed by treating the cells with forskolin (1 uM final concentration) followed immediately by test compound (6 concentrations) for an additional 10 min at 37°C. In separate experiments, 6 concentrations of antagonist were preincubated for 20 min prior to the addition of 10 nM 8-OH-DPAT and forskolin. The reaction was terminated by removal of the media and addition of 0.5 ml ice cold assay buffer. Plates were stored at -20°C prior to assessment of cAMP formation by a cAMP SPA assay (Amersham).

The compounds tested correspond to those prepared in Examples 1 to 20 below. The results of the pharmacological test procedure are set forth in Table I.

**Table I**

| **Example** | **5-HT**_{**1A**} **(Ki, nM)** | **ST (K**_{**i**}**, nM, % Inh. @.1 µM)** |
|---|---|---|
| 1 | 6.9 | 79 |
| 2 | 5.9 | 112 |
| 3 | 7.8 | 21 |
| 4 | 76.1 | 81 |
| 5 | 8.5 | 5.7 |
| 6 | 7.2 | 23 |
| 7 | 30.2 | 23 |
| 8 | 2.8 | 25 |
| 9 | 94.4 | 20 |
| 10 | 8.15 | 85 |
| 11 | 7.14 | 43 |
| 12 | 75.8 | 7 |
| 13 | 15.3 | 15 |
| 14 | 16.8 | 26 |
| 15 | 75.2 | 44 |
| 16 | 11.0 | 16 |
| 17 | 103.0 | 15 |
| 18 | 18.1 | 34 |
| 19 | 63.4 | 40 |
| 20 | 12.3 | 23 |

As demonstrated by the results set forth above, the compounds of the present invention are active towards 5-HT_{1A} receptors and generally elevate serotonin concentrations by inhibiting 5-HT transport. Accordingly, it is believed that the present compounds would be useful in treating disorders related to defects in serotonin concentration, such as anxiety and depression.

The compounds of this invention may be administered orally or parenterally, neat or in combination with conventional pharmaceutical carriers. Applicable solid carriers can include one or more substances which may also act as flavoring agents, lubricants, solubilizers, suspending agents, fillers, glidants, compression aids, binders or tablet-disintegrating agents or an encapsulating material. In powders, the carrier is a finely divided solid which is in admixture with the finely divided active ingredient. In tablets, the active ingredient is mixed with a carrier having the necessary compression properties in suitable proportions and compacted in the shape and size desired. The powders and tablets preferably contain up to 99% of the active ingredient. Any of the solid carriers known to those skilled in the art may be used with the compounds of this invention. Particularly suitable solid carriers include, for example, calcium phosphate, magnesium stearate, talc, sugars, lactose, dextrin, starch, gelatin, cellulose, methyl cellulose, sodium carboxymethyl cellulose, polyvinylpyrrolidone, low melting waxes and ion exchange resins.

Liquid carriers may be used in preparing solutions, suspensions, emulsions, syrups and elixirs of the compounds of this invention. The compounds of this invention can be dissolved or suspended in a pharmaceutically acceptable liquid carrier such as water, an organic solvent, a mixture of both or pharmaceutically acceptable oils or fat. The liquid carrier can contain other suitable pharmaceutical additives such as solubilizers, emulsifiers, buffers, preservatives, sweeteners, flavoring agents, suspending agents, thickening agents, colors, viscosity regulators, stabilizers or osmo-regulators. Suitable examples of liquid carriers for oral and parenteral administration include water (particularly containing additives as above, e.g., cellulose derivatives, preferably sodium carboxymethyl cellulose solution), alcohols (including monohydric alcohols and polyhydric alcohols e.g. glycols) and their derivatives and oils (e.g. fractionated coconut oil and arachis oil). For parenteral administration, the carrier can also be an oily ester such as ethyl oleate and isopropyl myristate. Sterile liquid carriers are used in sterile liquid form compositions for parenteral administration.

Liquid pharmaceutical compositions which are sterile solutions or suspensions can be utilized by, for example, intramuscular, intraperitoneal or subcutaneous injection. Sterile solutions can also be administered intravenously. Compositions for oral administration may be either liquid or solid composition form.

Preferably, the pharmaceutical compositions containing the compounds of this invention are in unit dosage form, e.g. as tablets or capsules. In such form, the compositions may be sub-divided in unit doses containing appropriate quantities of the present compounds. The unit dosage forms can be packaged compositions, for example, packeted powders, vials, ampoules, prefilled syringes or sachets containing liquids. Alternatively, the unit dosage form can be, for example, a capsule or tablet itself, or it can be the appropriate number of any such compositions in package form.

The therapeutically effective amount of the compounds of this invention that is administered and the dosage regimen depends on a variety of factors, including the weight, age, sex, and medical condition of the subject, the severity of the disease, the route and frequency of administration, and the specific compound employed, and thus may vary widely. However, it is believed that the pharmaceutical compositions may contain the compounds of this invention in the range of about 0.1 to about 2000 mg, preferably in the range of about 0.5 to about 500 mg and more preferably between about I and about 100 mg. Projected daily dosages of active compound are about 0.01 to about 100 mg/kg body weight. The daily dose can be conveniently administered two to four times per day.

The present invention may be embodied in other specific forms without departing from the spirit and essential attributes thereof and accordingly, reference should be made to the appended claims, rather than to the foregoing specification, as indicating the scope of the invention.

## Claims

1. A compound of the formula: 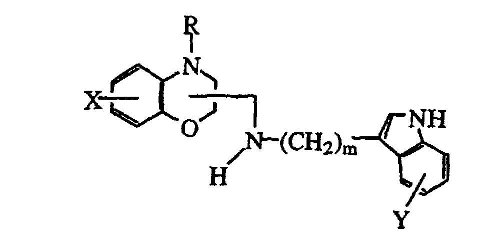 wherein:
R is hydrogen, alkyl of 1 to 6 carbon atoms, phenyl, benzyl or substituted phenyl;
X and Y are each, independently, hydrogen, halogen, cyano, or alkoxy of 1 to 6 carbon atoms; and
m is 1 to 5;
or a pharmaceutically acceptable salt thereof.

2. A compound as claimed in claim 1, wherein R is hydrogen, cyano or alkyl of 1 to 4 carbon atoms.

3. A compound as in claim 1 or claim 2 wherein X and Y are each, independently, hydrogen, halogen or cyano.

4. A compound as claimed in any one of claims 1 to 3, wherein m is 2 to 4.

5. A compound according to Claim 1 which is one of the following:
[(3,4)-dihydro-2H-benzo[1,4]oxazin-2-yl-methyl)]-[3-(1H-indol-3-yl)propyl]amine;
[3-(1H-indol-3-yl)-propyl]-(4-methyl-3,4-dihydro-2H-benzo[1,4]oxazin-2-ylmethyl)amine;
(4-benzyl-3,4-dihydro-2H-benzo[1,4]oxazin-2-yl-methyl)-[3-(1H-indol-3-yl)propyl]amine;
(4-benzyl-3,4-dihydro-2H-benzo[1,4]oxazin-2-yl-methyl)-[3-(1H-indo-3-yl)-butyl]-amine;
[3-(5-fluoro-1H-indol-3-yl)-propyl]-(4-methyl-3,4-dihydro-2H-benzo[1,4)oxazin-2-yl-methyl)-amine;
[3-(1H-indol-3-yl)-propyl]-(4-benzyl-3,4-dihydro-2H-benzo[1,4]oxazin-2-yl-methyl)-amine;
[3-(1H-indol-3-yl)-propyl]-(4-phenyl-3,4-dihydro-2H-benzo[1,4]oxazin-2-yl-methyl)-amine;
[3-(5-fluoro-1H-indo]-3-yl)-propyl]-4-isopropyl-3,4-dihydro-2H-benzo[1,4]oxazin-2-yl-methyl-amine;
[3-(1H-indol-3-yl)-butyl]-(4-isopropyl-3,4-dihydro-2H-benzo[1,4]oxazin-2-yl-methyl)-amine;
[3-(5-fluoro-lH-indol-3-yl)-propyl]-(4-isobutyl-3,4-dihydro-2H-benzo[1,4]oxazin-2-yl-methyl)-amine;
[3-(5-fluoro-1H-indol-3-yl)-propyl]-(4-phenyl-3,4-dihydro-2H-benzo[1,4]oxazin-2-yl-methyl)-amine;
[3-(5-fluoro-1H-indol-3-yl)-butyl]-(4-phenyl-3,4-dihydro-2H-benzo[1,4]oxazine-2-yl-methyl)-amine;
[3-(5-fluoro-lH-indol-3-yl)-propyl]-(4-ethyl-3,4-dihydro-2H-benzo[1,4]oxazin-2-yl-methyl)-amine;
[3-(5-fluoro-1H-indol-3-yl)-propyl]-(4-propyl-3,4-dihydro-2H-benzo[1,4]oxazine-2-yl-methyl)-amine;
(7-chloro-4-methyl-3,4-dihydro-2H-benzo[1,4]oxazin-2-yl-methyl)-[3-(5-fluoro-1H-indol-3-yl)-propyl]-propyl)-amine;
(3,4-dihydro-2H-benzo[1,4]oxazin-2-yl-methyl)-[3-(5-fluoro-1H-indol-3-yl)-propyl]-amine;
7-methoxy-3,4-dihydro-2H-benzo[1,4]oxazin-2-yl-methyl-[3-(5-fluoro-1H-indol-3-yl)-propyl]-amine;
[3-(5-fluoro-1H-indol-3-yl)-propyl]-4-phenyl-3,4-dihydro-2H-benzo[1,4]oxazin-2-yl-methyl);
or
(3,4-dihydro-2H-benzo[1,4]oxazin-3-yl-methyl)-[3-(5-fluoro-1H-indol-3-yl)propyl]-amine;
or a pharmaceutically acceptable salt thereof,

6. A pharmaceutical composition comprising a compound of the formula: 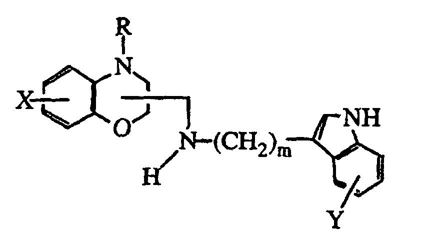 wherein:
R is hydrogen, alkyl of 1 to 6 carbon atoms, phenyl, benzyl or substituted phenyl;
X and Y are each, independently, hydrogen, halogen, cyano or alkoxy of 1 to 6 carbon atoms; and
m is 1 to 5; or
a pharmaceutically acceptable salt thereof and a pharmaceutically acceptable carrier.

7. A process for preparing a compound of formula I as defined in claim 1 or a pharmaceutically acceptable salt thereof, which comprises one of the following:
a) reducing a compound of formula II 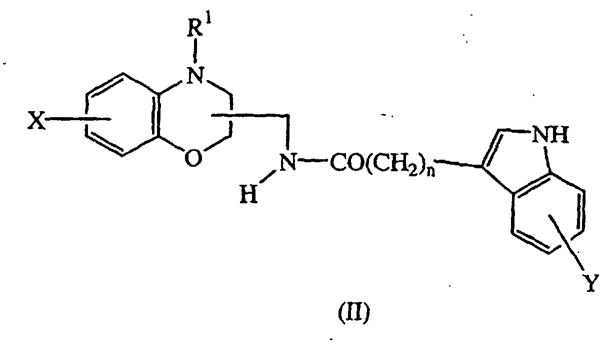 wherein n= 0 to 4, and X and Y are as defined in claim 1 and R¹ is R as defined in claim 1or R¹ is COO^{t}Bu to give a compound of formula I wherein m = n +1; or
b) reducing a compound of formula III 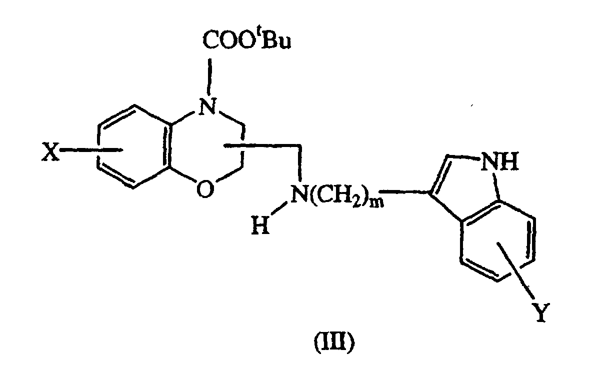 wherein m, X and Y are as defined in claim1 to give a corresponding compound of formula I wherein R is methyl;
or
c) reacting a compound of formula 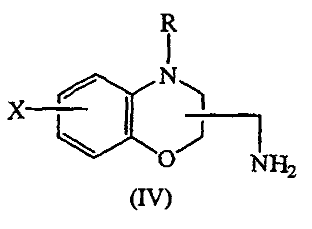 wherein R and X are as hereinbefore defined in claim 1,
with a compound of formula 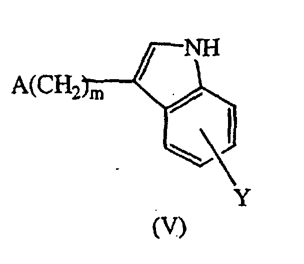 wherein m and Y are as hereinbefore defined and A is a leaving group selected from halogen or an organic sulphonyloxy group e.g aryl or alkylsulphonyloxy group such as tosyloxy;
or
d) reacting a compound of formula 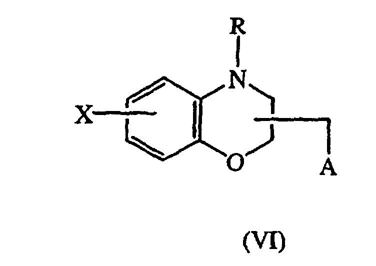 wherein X and R are as defined above and A is a leaving group as defined above, with a compound of formula 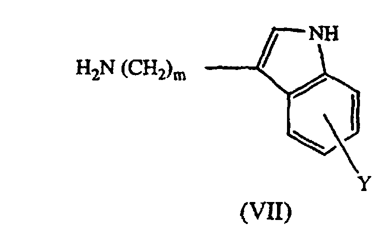 wherein m and Y are as hereinbefore defined;.
or
e) deprotecting a compound of formula 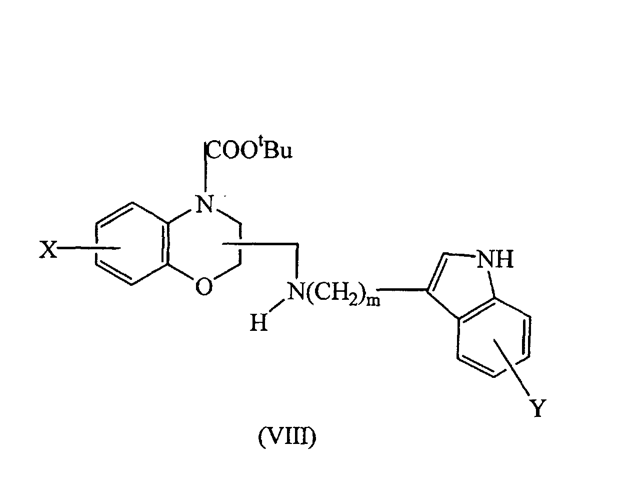 wherein m, X and Y are as defined in claim 1 to give a compound of formula I wherein R is hydrogen;
or
f) resolving a mixture (e.g racemate) of optically active isomers of a compound of formula I to isolate one enantiomer or diastereomer substantially free of the other enantiomer or diastereomers;
or
g) acidifying a basic compound of formula I with a pharmaceutically acceptable acid to give a pharmaceutically acceptable salt.

8. A compound as claimed in any one of Claims 1 to 5 for use as pharmaceutical.

9. Use of a compound according to any one of Claims 1 to 5 for the preparation of a medicament for alleviating the symptoms of depression.

## Patentansprüche

1. Verbindung der Formel 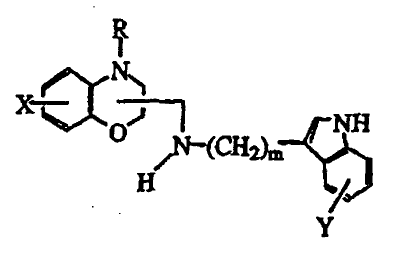 worin:
R Wasserstoff, Alkyl mit 1 bis 6 Kohlenstoffatomen, Phenyl, Benzyl oder substituiertes Phenyl darstellt;
X und Y jeweils unabhängig Wasserstoff, Halogen, Cyano oder Alkoxy mit 1 bis 6 Kohlenstoffatomen darstellen; und
m 1 bis 5 ist;
oder ein pharmazeutisch annehmbares Salz davon.

2. Verbindung wie in Anspruch 1 beansprucht, worin R Wasserstoff, Cyano oder Alkyl mit 1 bis 4 Kohlenstoffatomen darstellt.

3. Verbindung wie in Anspruch 1 oder Anspruch 2 beansprucht, worin X und Y jeweils unabhängig Wasserstoff, Halogen oder Cyano darstellen.

4. Verbindung wie in einem der Ansprüche 1 bis 3 beansprucht, worin m 2 bis 4 ist.

5. Verbindung gemäß Anspruch 1, welche eine der folgenden ist:
[(3,4)-Dihydro-2H-benzo[1,4]oxazin-2-yl-methyl)]-[3-(1H-indol-3-yl)propyl]amin;
[3-(1H-Indol-3-yl)-propyl]-(4-methyl-3,4-dihydro-2Hbenzo[1,4]oxazin-2-ylmethyl)amin;
(4-Benzyl-3,4-dihydro-2H-benzo[1,4]oxazin-2-ylmethyl)-[3-(1H-indol-3-yl)propyl]amin;
(4-Benzyl-3,4-dihydro-2H-benzo[1,4]oxazin-2-ylmethyl) - [3-(1H-indo-3-yl)-butyl]-amin;
[3-(5-Fluor-lH-indol-3-yl)-propyl]-(4-methyl-3,4-dihydro-2Hbenzo[1,4]oxazin-2-yl-methyl)-amin;
[3-(1H-Indol-3-yl)-propyl]-(4-benzyl-3,4-dihydro-2Hbenzo[1,4]oxazin-2-yl-methyl)-amin;
[3-(1H-Indol-3-yl)-propyl]-(4-phenyl-3,4-dihydro-2Hbenzo[1,4]oxazin-2-yl-methyl)-amin;
[3-(5-Fluor-lH-indol-3-yl)-propyl]-4-isopropyl-3,4-dihydro-2H-benzo[1,4]oxazin-2-yl-methyl-amin;
[3-(1H-indol-3-yl)-butyl]-(4-isopropyl-3,4-dihydro-2Hbenzo[1,4]oxazin-2-yl-methyl)-amin;
[3-(5-Fluor-lH-indol-3-yl)-propyl]-(4-isobutyl-3,4-dihydro-2H-benzo[1,4]oxazin-2-yl-methyl)-amin;
[3-(5-Fluor-1H-indol-3-yl)-propyl]-(4-phenyl-3,4-dihydro-2Hbenzo[1,4]oxazin-2-yl-methyl)-amin;
[3-(5-Fluor-lH-indol-3-yl)-butyl]-(4-phenyl-3,4-dihydro-2Hbenzo[1,4]oxazin-2-yl-methyl)-amin;
[3-(5-Fluor-lH-indol-3-yl)-propyl]-(4-ethyl-3,4-dihydro-2Hbenzo[1,4]oxazin-2-yl-methyl)-amin;
[3-(5-Fluor-lH-indol-3-yl)-propyl]-(4-propyl-3,4-dihydro-2Hbenzo[1,4]oxazin-2-yl-methyl)-amin; (7-Chlor-4-methyl-3,4-dihydro-2H-benzo[1,4]oxazin-2-yl-methyl)-[3-(5-fluor-lH-indol-3-yl)-propyl]-propyl)-amin;
(3,4-Dihydro-2H-benzo[1,4]oxazin-2-yl-methyl)-[3-(5-fluor-1H-indol-3-yl)-propyl]-amin;
7-Methoxy-3,4-dihydro-2H-benzo[1,4]oxazin-2-yl-methyl-[3-(5-fluor-1H-indol-3-yl)-propyl]-amin;
[3-(5-Fluor-lH-indol-3-yl)-propyl]-4-phenyl-3,4-dihydro-2Hbenzo[1,4]oxazin-2-yl-methyl);
oder
(3,4-Dihydro-2H-benzo[1,4]oxazin-3-yl-methyl)-[3-(5-fluor-1H-indol-3-yl)propyl]-amin;
oder ein pharmazeutisch annehmbares Salz davon.

6. Pharmazeutische Zusammensetzung, welche ein Verbindung der Formel: 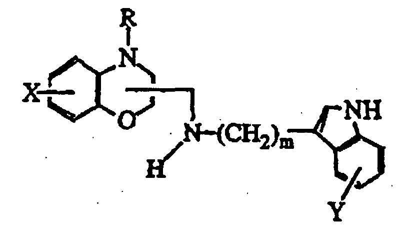 worin:
R Wasserstoff, Alkyl mit 1 bis 6 Kohlenstoffatomen, Phenyl, Benzyl oder substituiertes Phenyl darstellt;
X und Y jeweils unabhängig Wasserstoff, Halogen, Cyano oder Alkoxy mit 1 bis 6 Kohlenstoffatomen darstellen; und
m 1 bis 5 ist;
oder ein pharmazeutisch annehmbares Salz davon und einen pharmazeutisch annehmbaren Träger umfasst.

7. Verfahren zum Herstellen einer Verbindung der Formel I, wie in Anspruch 1 definiert, oder eines pharmazeutisch annehmbaren Salzes davon, welches eines der folgenden umfasst:
a) Reduzieren einer Verbindung der Formel II 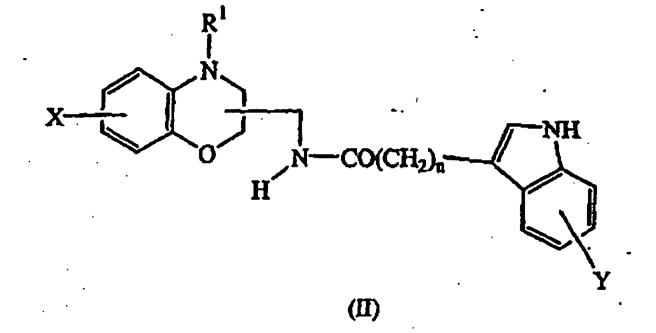 worin n = 0 bis 4 und X und Y wie in Anspruch 1 definiert sind und R¹ für R steht, wie in Anspruch 1 definiert, oder R¹ für COO^{t}Bu steht, um eine Verbindung der Formel I zu ergeben, worin m = n+1; oder
b) Reduzieren einer Verbindung der Formel III 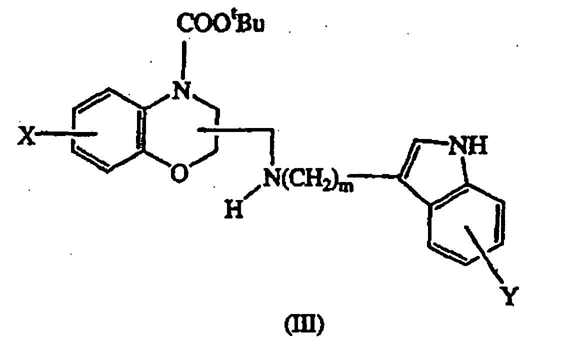 worin m, X und Y wie in Anspruch 1 definiert sind, um eine entsprechende Verbindung der Formel I zu ergeben, worin R für Methyl steht;
oder
c) Umsetzen einer Verbindung der Formel 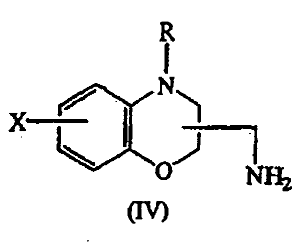 worin R und X wie hierin vorher in Anspruch 1 definiert sind, mit einer Verbindung der Formel 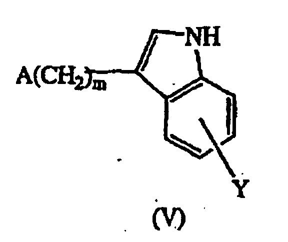 worin m und Y wie hierin vorher definiert sind und A eine Abgangsgruppe darstellt, ausgewählt aus Halogen oder einer organischen Sulfonyloxygruppe, z.B. Aryl- oder Alkylsulfonyloxygruppe, wie Tosyloxy;
oder
d) Umsetzen einer Verbindung der Formel 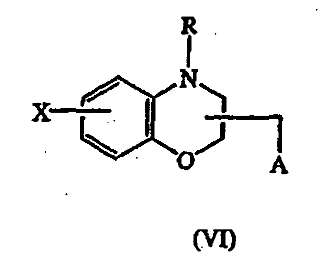 worin X und R wie oben definiert sind und A eine Abgangsgruppe darstellt, wie oben definiert,
mit einer Verbindung der Formel 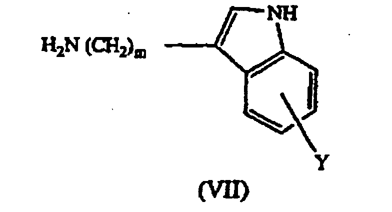 worin m und Y wie hierin vorher definiert sind;
oder
e) Entschützen einer Verbindung der Formel 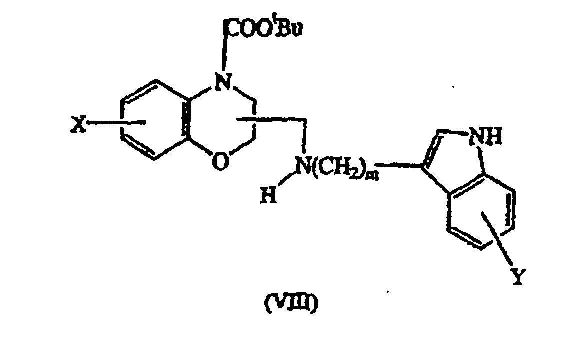 worin m, X und Y wie in Anspruch 1 definiert sind, um eine Verbindung der Formel I zu ergeben, worin R Wasserstoff darstellt; oder
f) Trennen eines Gemisches (z.B. Racemat) aus optisch aktiven Isomeren einer Verbindung der Formel I, um ein Enantiomer oder Diastereomer im Wesentlichen frei vom anderen Enantiomer oder Diastereomer zu isolieren;
oder
g) Ansäuern einer basischen Verbindung der Formel I mit einer pharmazeutisch annehmbaren Säure, um ein pharmazeutisch annehmbares Salz zu ergeben.

8. Verbindung wie in einem der Ansprüche 1 bis 5 beansprucht, zur Verwendung als Pharmazeutikum.

9. Verwendung einer Verbindung gemäß einem der Ansprüche 1 bis 5 für die Herstellung eines Medikaments zum Lindern der Symptome von Depression.

## Revendications

1. Composé de formule : 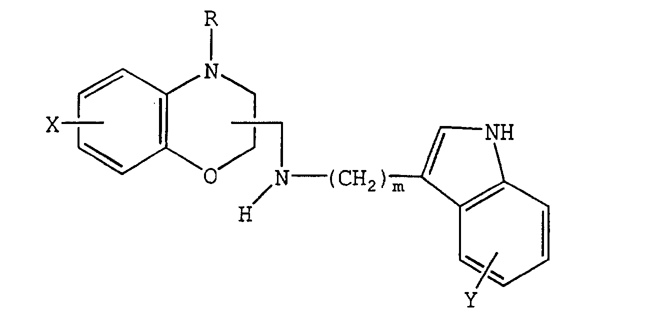 dans laquelle :
R est un hydrogène, un alkyle de 1 à 6 atomes de carbone, un phényle, un benzyle ou un phényle substitué;
X et Y sont chacun indépendamment un hydrogène, un halogène, un cyano, ou un alcoxy de 1 à 6 atomes de carbone; et
m est 1 à 5;
ou un sel pharmaceutiquement acceptable de celui-ci.

2. Composé suivant la revendication 1, dans lequel R est un hydrogène, un cyano ou un alkyle de 1 à 4 atomes de carbone.

3. Composé suivant la revendication 1 ou la revendication 2, dans lequel X et Y sont chacun indépendamment un hydrogène, un halogène ou un cyano.

4. Composé suivant l'une quelconque des revendications 1 à 3, dans lequel m est 2 à 4.

5. Composé suivant la revendication 1, qui est l'un des suivants :
la [(3,4)-dihydro-2H-benzo[1,4]oxazin-2-ylméthyl)]-[3-(1H-indol-3-yl)propyl]amine;
la [3-(1H-indol-3-yl)propyl]-(4-méthyl-3,4-dihydro-2H-benzo[1,4]oxazin-2-ylméthyl)amine;
la (4-benzyl-3,4-dihydro-2H-benzo[1,4]oxazin-2-yl-méthyl)-[3-(1H-indol-3-yl)propyl]amine;
la (4-benzyl-3,4-dihydro-2H-benzo[1,4]oxazin-2-yl-méthyl)-[3-(1H-indol-3-yl)butyl]amine;
la [3-(5-fluoro-lH-indol-3-yl)propyl]-(4-méthyl-3,4-dihydro-2H-benzo[1,4]oxazin-2-ylméthyl)aminé;
la [3-(1H-indol-3-yl)propyl]-(4-benzyl-3,4-dihydro-2H-benzo [1,4]oxazin-2-ylméthyl)amine;
la [3-(lH-indol-3-yl)propyl]-(4-phényl-3,4-dihydro-2H-benzo[1,4]oxazin-2-ylméthyl)amine;
la [3-(5-fluoro-1H-indol-3-yl)propyl]-4-i-propyl-3,4-dihydro-2H-benzo[1,4]oxazin-2-ylméthylamine;
la [3-(1H-indol-3-yl)butyl]-(4-i-propyl-3,4-dihydro-2H-benzo [1,4]oxazin-2-ylméthyl)amine;
la [3-(5-fluoro-lH-indol-3-yl)propyl]-(4-i-butyl-3,4-dihydro-2H-benzo[1,4]oxazin-2-ylméthyl)amine;
la [3-(5-fluoro-lH-indol-3-yl)propyl]-(4-phényl-3,4-dihydro-2H-benzo[1,4]oxazin-2-ylméthyl)amine;
la [3-(5-fluoro-1H-indol-3-yl)butyl]-(4-phényl-3,4-dihydro-2H-benzo[1,4]oxazin-2-ylméthyl)amine;
la [3-(5-fluoro-lH-indol-3-yl)propyl]-(4-éthyl-3,4-dihydro-2H-benzo[1,4]oxazin-2-ylméthyl)amine;
la [3-(5-fluoro-lH-indol-3-yl)propyl]-(4-propyl-3,4-dihydro-2H-benzo[1,4]oxazin-2-ylméthyl)amine;
la (7-chloro-4-méthyl-3,4-dihydro-2H-benzo[1,4]oxazin-2-ylméthyl)-[3- (5-fluoro-lH-indol-3-yl)propyl]propyl)-amine;
la (3,4-dihydro-2H-benzo[1,4]oxazin-2-ylméthyl)-[3-(5-fluoro-lH-indol-3-yl)propyl]amine;
la 7-méthoxy-3,4-dihydro-2H-benzo[1,4]oxazin-2-ylméthyl-[3-(5-fluoro-1H-indol-3-yl)propyl]amine;
la [3-(5-fluoro-lH-indol-3-yl)propyl]-4-phényl-3,4-dihydro-2H-benzo[1,4]oxazin-2-ylméthyle) ;
ou la (3,4-dihydro-2H-benzo[1,4]oxazin-3-ylméthyl)-[3-(5-fluoro-lH-indol-3-yl)propyl]amine;
ou un sel pharmaceutiquement acceptable de celui-ci.

6. Composition pharmaceutique comprenant un composé de formule : 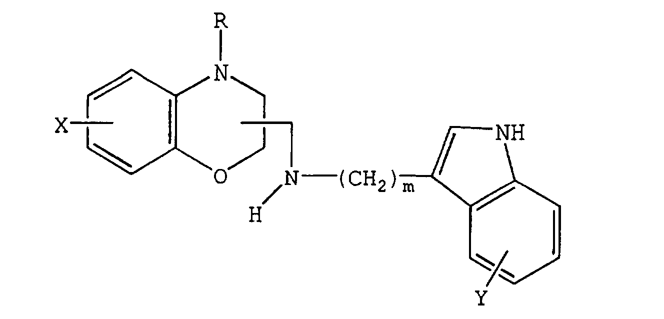 dans laquelle :
R est un hydrogène, un alkyle de 1 à 6 atomes de carbone, un phényle, un benzyle ou un phényle substitué;
X et Y sont chacun indépendamment un hydrogène, un halogène, un cyano, ou un alcoxy de 1 à 6 atomes de carbone; et
m est 1 à 5; ou
un sel pharmaceutiquement acceptable de celui-ci.

7. Procédé pour la préparation d'un composé de formule I suivant la revendication 1 ou d'un sel pharmaceutiquement acceptable de celui-ci, qui comprend l'une des étapes suivantes :
a) la réduction d'un composé de formule II 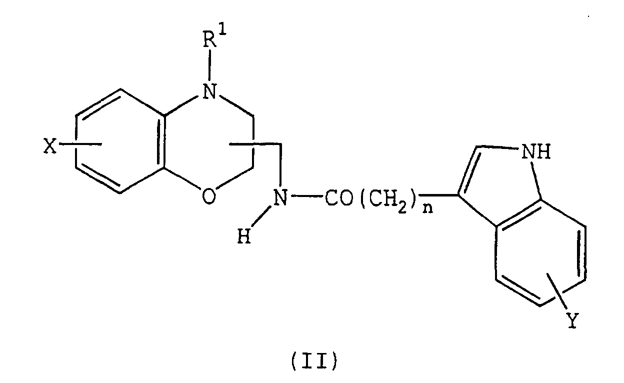 dans laquelle n=0 à 4, et X et Y sont comme définis à la revendication 1 et R¹ est R comme défini à la revendication 1 ou R¹ est COO^{t}Bu pour donner un composé de formule I dans lequel m = n+1; ou
b) la réduction d'un composé de formule III 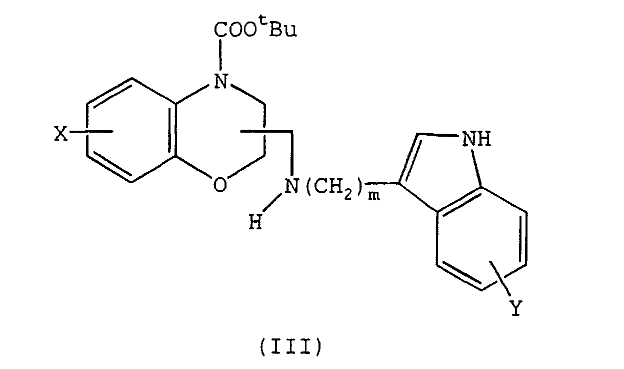 dans laquelle m, X et Y sont comme définis à la revendication 1 pour donner un composé correspondant de formule I dans laquelle R est un méthyle;
ou
c) la réaction d'un composé de formule 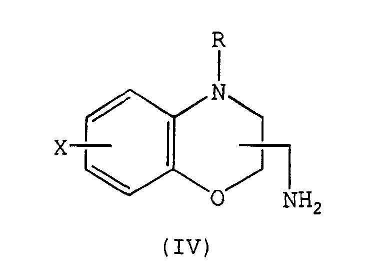 dans laquelle R et X sont comme définis ci-dessus dans le présent mémoire à la revendication 1,
avec un composé de formule 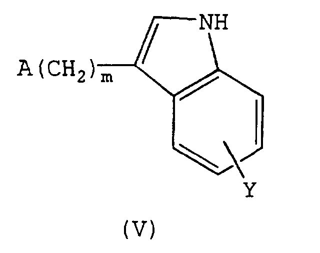 dans laquelle m et Y sont comme définis ci-dessus dans le présent mémoire et A est un groupement partant sélectionné parmi un halogène ou un groupement sulfonyloxy organique par exemple un groupement aryl- ou alkylsulfonyloxy tel qu'un tosyloxy;
ou
d) la réaction d'un composé de formule 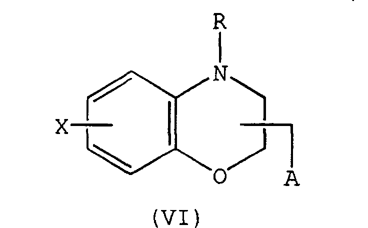 dans laquelle X et R sont comme définis ci-dessus et A est un groupement partant comme défini ci-dessus avec un composé de formule 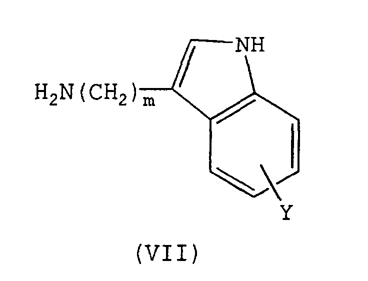 dans laquelle m et Y sont comme définis ci-dessus dans le présent mémoire;
ou
e) la déprotection d'un composé de formule 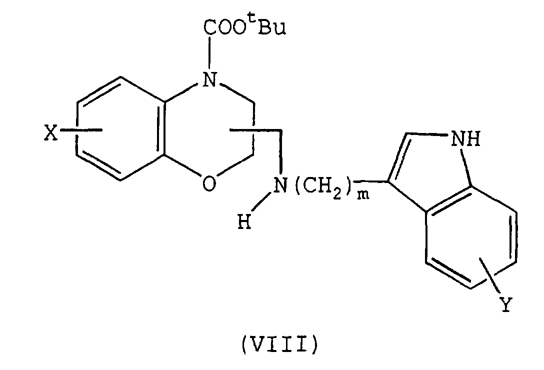 dans laquelle m, X et Y sont comme définis à la revendication 1 pour donner un composé de formule I dans laquelle R est un hydrogène;
ou
f) la résolution d'un mélange (par exemple un mélange racémique) d'isomères optiquement actifs d'un composé de formule I pour isoler un énantiomère ou un diastéréoisomère substantiellement exempt de l'autre énantiomère ou d'autres diastéréoisomères;
ou
g) l'acidification d'un composé basique de formule I avec un acide pharmaceutiquement acceptable pour donner un sel pharmaceutiquement acceptable.

8. Composé suivant l'une quelconque des revendications 1 à 5, pour une utilisation comme agent pharmaceutique.

9. Utilisation d'un composé suivant l'une quelconque des revendications 1 à 5, pour la préparation d'un médicament pour soulager les symptômes d'une dépression.
